# EUROPEAN PATENT APPLICATION

(11) **EP 4 530 598 A1**
(43) Date of publication of application: **02.04.2025**
(21) Application number: 23201060.3
(22) Date of filing: 29.09.2023

(54) **BIOMARKERS FOR GASTROINTESTINAL DISEASES IN ANIMALS**

(71) Applicant: Impextraco NV, 2220 Heist-Op-Den-Berg (BE)
(72) Inventor: The designation of the inventor has not yet been filed
(74) Representative: Brantsandpatents bv

(57) **Abstract**

The current invention relates to a method for predicting, diagnosing and/or evaluating a treatment of a gastrointestinal disease and/or disorder in an animal, preferably a monogastric animal such as a chicken, said method comprises the steps of analysing a collected biological sample from said animal for one or more biomarkers chosen from Table 1. The invention also relates to a method for evaluating the efficacy of a compound to prevent or treat a gastrointestinal disease and/or disorder in an animal, said method comprises administering to an animal an amount of said compound, and analysing one or more biomarkers chosen from Table 1; to a biomarker panel; to an immunoassay; and to a use of a biomarker panel.

## Description

### FIELD OF THE INVENTION

The present invention pertains to predicting, diagnosing, and/or evaluating a treatment of a gastrointestinal disease and/or disorder in an animal, preferably a monogastric animal such as a chicken, said method comprises the steps of analysing a collected biological sample from said animal for one or more biomarkers; and to a method for evaluating the efficacy of a compound to prevent or treat a gastrointestinal disease and/or disorder in an animal, said method comprises administering to an animal an amount of said compound, and analysing one or more biomarkers. Also, the present invention relates to peptide or protein blood serum biomarkers that can be detected by immunoassay.

### BACKGROUND

Intestinal health is of vital importance to the performance and wellbeing of (farm) animals. Although there is a clear relationship between animal performance and a healthy gastrointestinal tract, there is no clear definition for gut health. A healthy gut is based on the physiological homeostasis of different processes including nutrient digestion and absorption, host metabolism and energy generation, stable gut microbiome, mucus layer development, mucosal immune responses, and intestinal barrier function. The gastrointestinal tract is responsible for regulating this physiological homeostasis that provides the animal the ability to withstand endogenous and exogenous stressors, such as high feed intake, certain feed ingredients, specific pathogens, and toxins.

In poultry, dysbiosis, intestinal barrier leakage and intestinal inflammation have become major issues especially since the ban on antimicrobial growth promoters (AGP) in animal feed in the European Union in 2006 [Regulation (EC) N° 1831/2003]. Prior to that date, it was thought that intestinal health issues were largely kept under control by the widespread use of AGP. In order to maintain animal resilience in the post-AGP era, dietary additives are used to stimulate gut health. The number and diversity of additives available for animal feed have enormously increased during the last decades. Providing reliable information is essential for potential users of these products to evaluate the efficacy of feed additive. However, monitoring the efficacy of a selected feed additive on gut health in general in the process of product development or in-field product efficacy evaluation is complex.

What is more, the diversity of intestinal health problems and the broad variety of modes of action of animal feed additives have to be taken into account. Therefore, there is a clear need for easy to measure generic biomarkers independent of the endogenous or exogenous stressors in order to evaluate the efficacy of different types of feed additives for gut health stimulation.

The present invention aims to provide a solution for at least one of the problems identified above.

### SUMMARY OF THE INVENTION

The present invention and embodiments thereof serve to provide a solution to one or more of above-mentioned disadvantages. Biomarkers obtainable for blood serum have been identified that are representative for the intestinal health status in animals. These protein biomarkers allowed the development of an immunoassay for detection and use these biomarkers to measure the efficacy of feed additives/compositions on improving the intestinal health.

To this end, the present invention relates to a method for predicting, diagnosing, and/or evaluating a treatment of a gastrointestinal disease and/or disorder in an animal according to claim 1. In particular, the method comprises the steps of analysing a collected biological sample from said animal for one or more biomarkers chosen from Table 1.

The invention also relates to method for evaluating the efficacy of a compound to prevent or treat a gastrointestinal disease and/or disorder in an animal according to claim 12. In particular, said method comprises administering to an animal an amount of said compound, and analysing one or more biomarkers chosen from Table 1.

The method further also relates to a biomarker panel for predicting, diagnosing, and/or evaluating a treatment of a gastrointestinal disease and/or disorder in an animal according to claim 14; to an immunoassay for predicting, diagnosing, and/or evaluating a treatment of a gastrointestinal disease and/or disorder in an animal according to claim 15; and to a use of a biomarker panel for predicting, diagnosing, and/or evaluating a treatment of a gastrointestinal disease and/or disorder in an animal according to claim 16.

### DESCRIPTION OF FIGURES

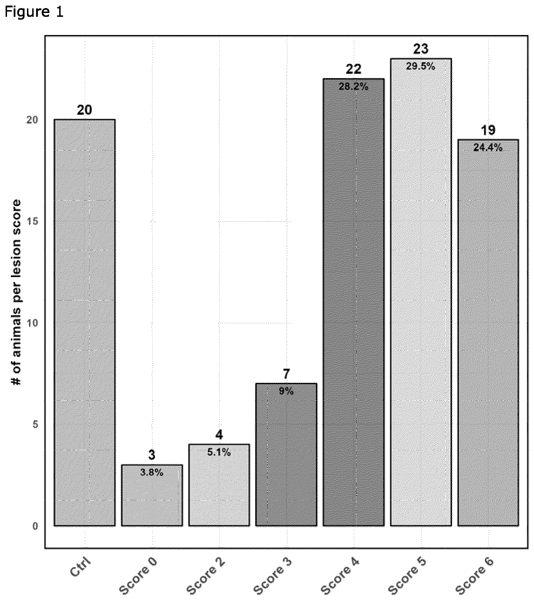

**Figure 1** shows the distribution of the necrotic lesion scores from birds derived from a necrotic enteritis in vivo trial. The total lesion score/bird was calculated as average NE-score from three middle parts of the small intestinal segments. The graph represents the number of birds corresponding to the lesion score from birds challenged with C. perfringens resulting in varying severity of the disease: no necrotic lesions (score 0; n=3); mild intestinal necrosis (score 2; n=4); moderate necrotic enteritis (score 3 and 4; n= 7 and 22, respectively) or severe necrosis (score 5 and 6; n=23 and 19, respectively). The number of animals that were not challenged with C. perfringens (Ctrl) and were used as negative control group, was equal to n=4. The percentage value corresponds to a calculated fraction of the total number of birds challenged with C. perfringens.

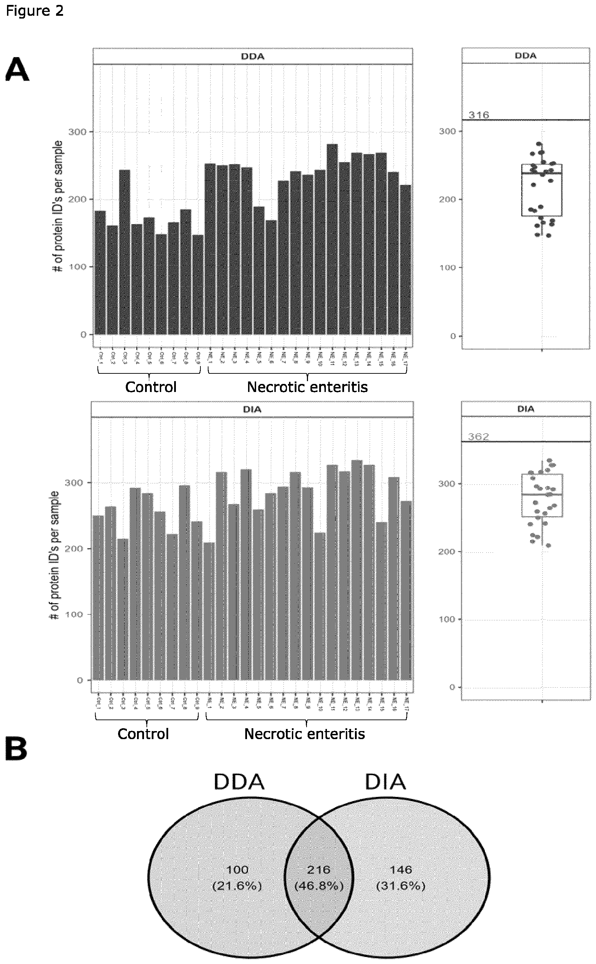

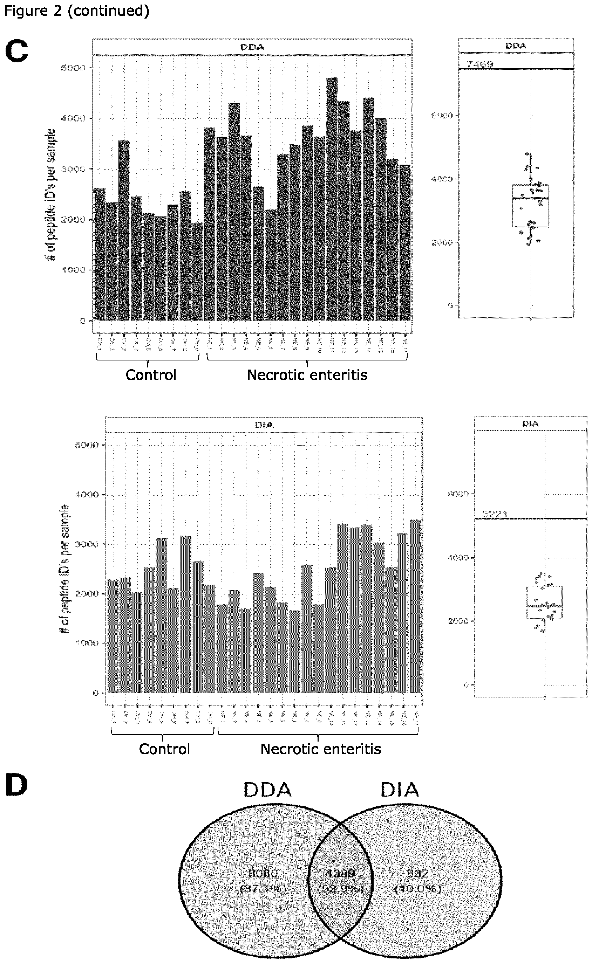

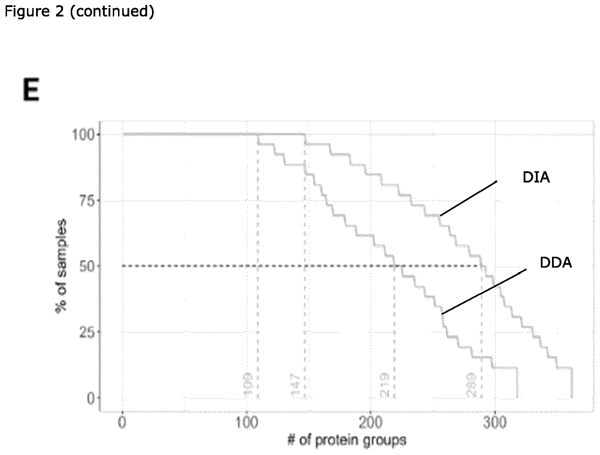

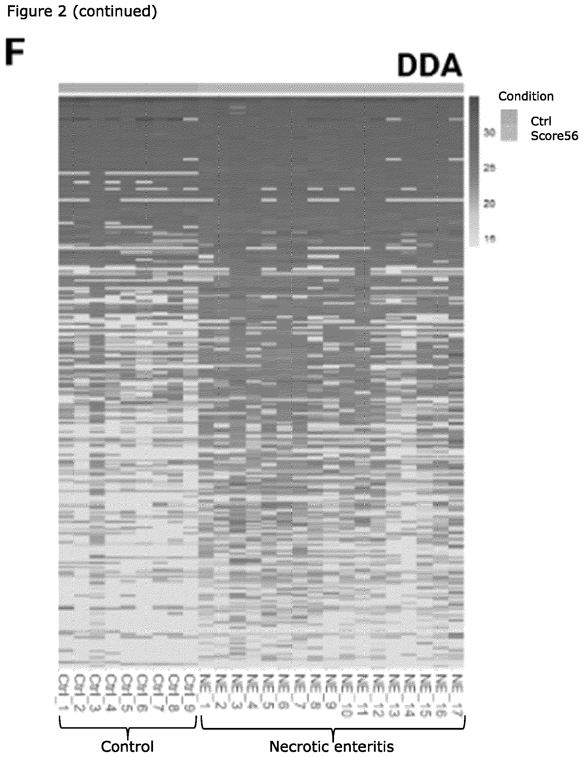

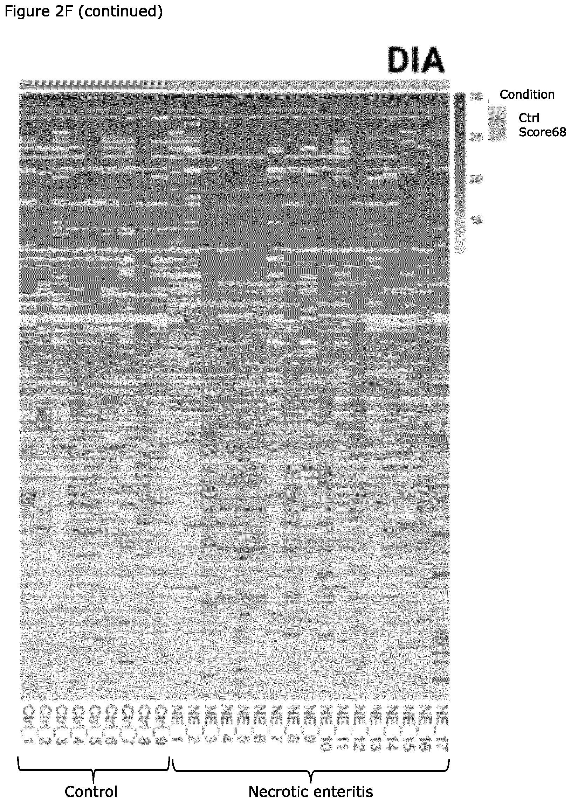

**Figure 2** shows an analysis of the data output. **Figure 2A** shows bar plots indicating the numbers of identified proteins per sample. The upper graph represent the DDA results while the lower graph represents DIA acquisitions. The boxplots of protein ID distributions show total number of proteins, median (centre line), interquartile range (IQR) and the whiskers as multiple of 1.5 * IQR. **Figure 2B** shows a Venn diagram showing the number and overlap of identified protein IDs between DDA and DIA methods. A total of 462 unique protein IDs were identified with both methods, 216 of which (46.8 %) were common for both DDA and DIA. **Figure 2C** shows bar plots indicating the numbers of identified peptides per sample. The upper graph represents DDA results, while the lower graph represents DIA results. The boxplots of protein ID distributions show total number of peptides, median (centre line), interquartile range (IQR) and the whiskers as multiple of 1.5 * IQR. **Figure 2D** shows a Venn diagram showing the number and overlap of identified peptides between DDA and DIA methods. A total of 8301 unique precursor peptides were identified with both methods, 4389 of which (52.9 %) were common for DDA and DIA. **Figure 2E** is a plot of data completeness. Numbers of fully quantified protein groups with an increasing percentage of samples identifies with DDA (lower) and DIA (upper) in chicken plasma proteome. Fractions of fully quantified proteins at 50% and 100% data completeness are indicated. **Figure 2F****:** Heatmap representation of intensities corresponding to quantified proteins in each sample. Low abundant proteins are coloured yellow (lighter), high abundant proteins are coloured blue (darker), fractions of missing values are indicated in grey. Most of the missing data accumulated around the proteins of low intensity, spread differently across the acquisitions of each method.

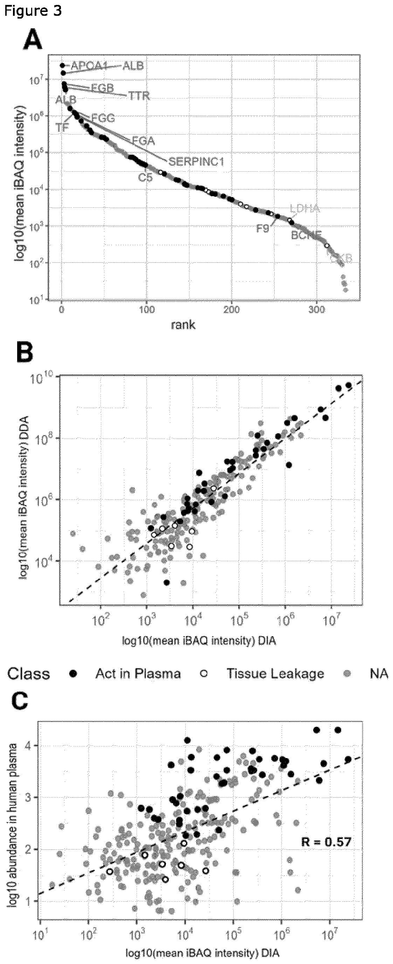

**Figure 3** shows a comparison of the chicken and human blood plasma proteomes. **Figure 3A****:** Scatter plot of chicken plasma proteins ranked according to relative abundance. **Figure 3B****:** Similarity between protein quantification in DIA (x-axis) and DDA (y-axis). **Figure 3C****:** Comparison of protein abundances in human and chicken blood plasma (n=265). Human protein data come from the latest build of the Human Plasma PeptideAtlas.

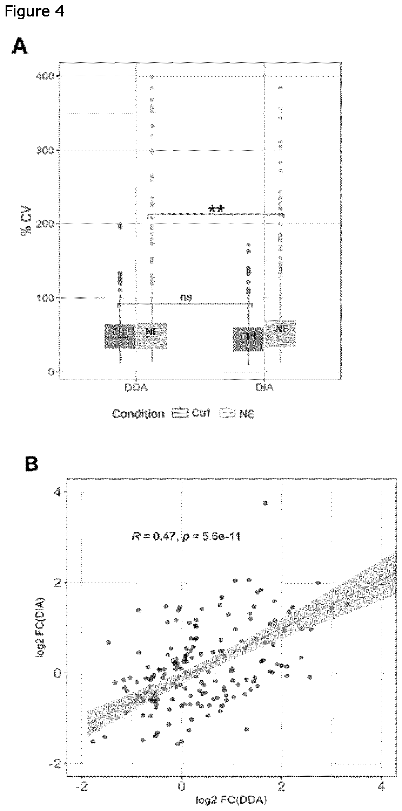

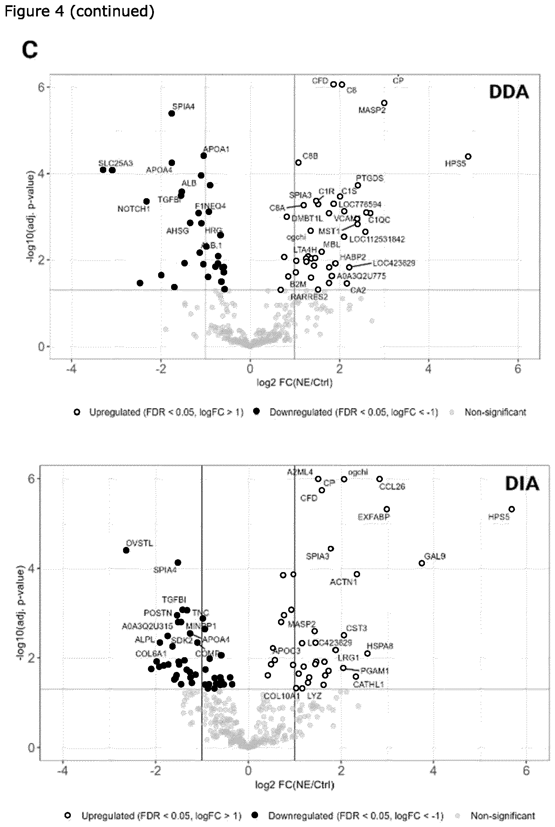

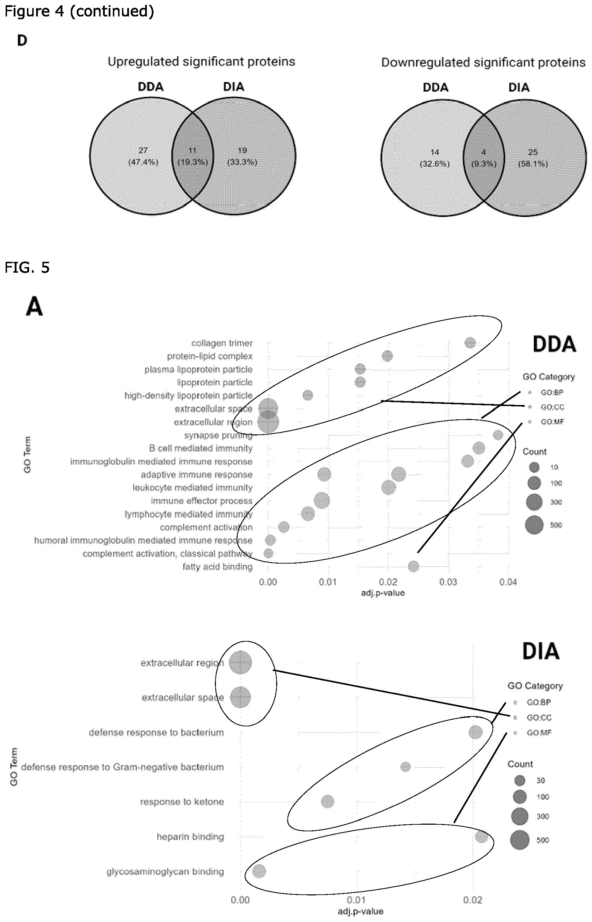

Figure 4 shows an analysis of the data output. **Figure 4A****:** Boxplots showing the distribution of the coefficients of variation (CV, %) of normalized LFQ intensities for the proteins (n = 175) common between the two methods, in all analytical samples. A Kolmogorov-Smirnov (KS) test was performed between the common protein intensities for DDA and DIA per experimental condition. CV values were computed as a ratio of the standard deviation of the protein intensity to the mean. For both methods, overall shift to higher CV values was found for two challenge groups, with values fluctuating around 50%. No statistically significant difference was detected between protein intensities found in control group analysed with DDA and DIA (blue boxplots, n = 9); significant variation between intensities of the common protein within NE-group (yellow boxplots, n = 17) was observed (KS test, p-value = 3.124 e-9). The two asterisks indicate that the p-value obtained from KS-test was lower than 0.01. **Figure 4B****:** Scatter plot of expressions, represented as log2 (NE/control) fold change (FC) values, of the common proteins (n =175), defined by each method. Middling correlation (Spearman correlation test, R= 0.47, p =5.6e-11) confirms presence of quantification accuracy and confidence between DDA and DIA methods. **Figure 4C****:** Volcano plots showing detected significantly (adj. p-value < 0.05, fold-change |log2FC| ≥ 1) differently abundant plasma proteins between the control and NE animals, analysed with DDA (left) and DIA (right). Significance cut-offs are indicated by lines. **Figure 4D****:** Venn diagrams showing the number and overlap of significant proteins that are differentially expressed in the two experimental conditions. A total of 57 unique proteins were identified as upregulated (left) identified with DDA and DIA, 11 of which were common for the two methods. A total of 43 unique proteins were identified as downregulated (right), 4 of which were common between DDA and DIA.
**Figure 5** shows results of a shotgun proteomics and gene ontology (GO) analysis. **Figure 5A****:** Bubble plot visualizing significantly enriched GO-terms for the differentially expressed proteins discovered upon the shotgun DDA (top) and DIA (bottom) proteomics analysis. GO analysis was performed using g:Profiler on the lists of Protein IDs corresponding to differentially regulated proteins The lists were divided according to the GO-categories: biological processes (BP), cellular components (CC), and molecular functions (MF). Size of the bubbles is proportional to the gene count (adj. p value < 0.05) assigned to the GO term, while circles represents GO category. The x-axis represents the adjusted p-value for the GO terms, and the y-axis displays the GO term. The results confirm that the majority of significant enriched genes belong to BP-category and vastly relate to processes of immune defence (DDA) or responses to bacteria (DIA), although the highest gene count was detected within CC-category (extracellular space and extracellular region), significantly enriched within regulated proteins discovered by both DDA and DIA methods.

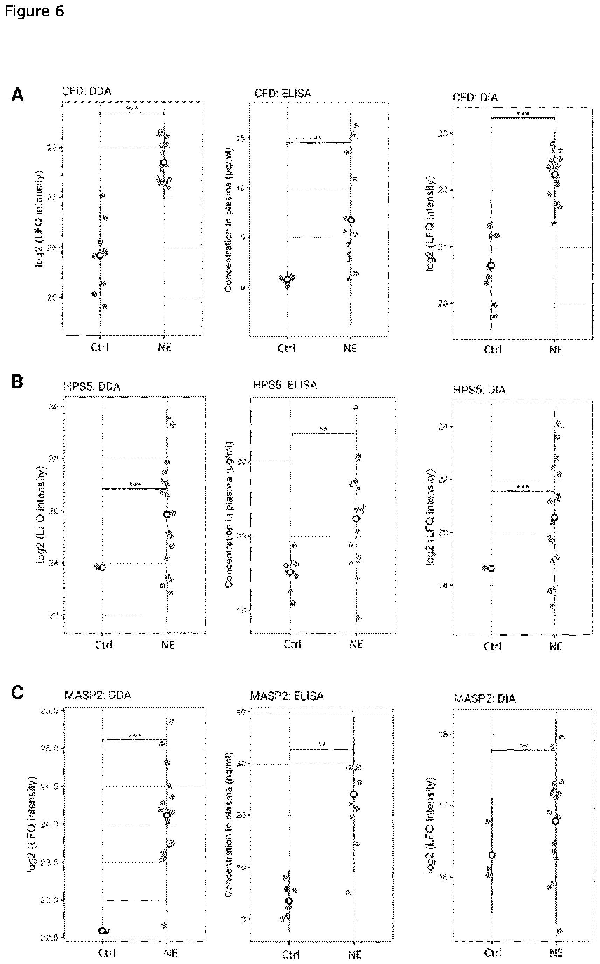

**Figure 6** shows protein abundances in blood plasma of birds detected with mass spectrometry-based proteomic analysis and ELISA immunoassay. Plasma levels of complement factor D (CFD - **Figure 6A****),** serum amyloid protein (HPS5 - **Figure 6B****),** and mannan binding lectin associated serine protease (MASP2 - **Figure 6C****)** were measured in non-infected and infected chickens. Each dot corresponds to one animal from experimental condition with a total of 9 birds from the control (left, n = 9) and 16 - from the challenge (right, n = 16) groups. Each row represents one of analysed proteins (A- CFD, B- HPS5, C- MASP2), each column corresponds to the method the protein was detected with (left - DDA, middle - ELISA, right- DIA). Results of ELISA are presented as the average of duplicate measurements. Asterisks *, ** and *** denote statistical significance of p < 0.05, p <0.01, p <0.001 between control and challenge group, respectively.

### DETAILED DESCRIPTION OF THE INVENTION

The present invention concerns a method for predicting, diagnosing, and/or evaluating a treatment of a gastrointestinal disease and/or disorder in an animal, said method comprises the steps of analysing a collected biological sample from said animal for one or more biomarkers chosen from Table 1; and further to methods for evaluating the efficacy of a compound to prevent or treat a gastrointestinal disease and/or disorder in an animal, said method comprises administering to an animal an amount of said compound, and analysing one or more biomarkers chosen from Table 1. The invention also concerns a biomarker panel and an immunoassay for predicting, diagnosing, and/or evaluating a treatment of a gastrointestinal disease and/or disorder in an animal, preferably a monogastric animal such as a chicken, wherein said biomarker panel comprises at least two markers chosen from Table 1.

The inventors surprisingly found that biomarkers of Table 1 are particularly indicative of a gastrointestinal disease and/or disorder in an animal, and are therefore useful in predicting, diagnosing, and/or evaluating a treatment of such disease and/or disorder.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the following terms have the following meanings:
"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed invention. However, it is to be understood that the value to which the modifier "about" refers is itself also specifically disclosed.

"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

Furthermore, the terms first, second, third and the like in the description and in the claims, are used for distinguishing between similar elements and not necessarily for describing a sequential or chronological order, unless specified. It is to be understood that the terms so used are interchangeable under appropriate circumstances and that the embodiments of the invention described herein are capable of operation in other sequences than described or illustrated herein.

The recitation of numerical ranges by endpoints includes all numbers and fractions subsumed within that range, as well as the recited endpoints.

The expression "% by weight", "weight percent", "%wt" or "wt%", here and throughout the description unless otherwise defined, refers to the relative weight of the respective component based on the overall weight of the formulation.

Whereas the terms "one or more" or "at least one", such as one or more or at least one member(s) of a group of members, is clear *per se,* by means of further exemplification, the term encompasses *inter alia* a reference to any one of said members, or to any two or more of said members, such as, e.g., any ≥3, ≥4, ≥5, ≥6 or ≥7 etc. of said members, and up to all said members.

Unless otherwise defined, all terms used in disclosing the invention, including technical and scientific terms, have the meaning as commonly understood by one of ordinary skill in the art to which this invention belongs. By means of further guidance, definitions for the terms used in the description are included to better appreciate the teaching of the present invention. The terms or definitions used herein are provided solely to aid in the understanding of the invention.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

The role of proteins, their properties, functions and localization in the living organisms has been intensively studied for decades. Owing to the importance of these molecules in the cellular morphogenesis and organization, alterations in protein expression or protein misplacement within an organism can correlate with health and, potentially, disease and its progression. Changes in protein expression profiles in a biological matrix such as blood has been associated with disease conditions and can be used as health biomarkers.

Biomarkers have emerged as competitive and prominent alternatives to 'golden standard testing methods in medical diagnostics. A biomarker has been defined as a biological substance that can be objectively measured and analysed and serves as an indicator of normal physiological or pathological processes, or pharmacologic responses to a therapeutic intervention. A bridge between the observed clinical effect and the organism's response can be built by evaluating the level of biomarkers in biological matters, and this will enable sound justification of the empirical finding.

In conventional veterinary medicine, blood analysis has been a canonical method of diagnostics, prognostics and health measurement. Compared to human medicine, untargeted discovery of novel blood-based biomarkers in veterinary research remains at its dawn, although the overall engagement of so-called proteomic technologies in the field is increasing. Proteomics technologies are high-throughput approaches that can identify and quantify the collection of proteins in biological samples, also called the proteome. Several approaches exist, of which mass spectrometry (MS)-based proteomics has evolved to a standard technology for untargeted discovery, including the detection of novel protein biomarkers in plasma or serum.

In animal production, health biomarkers are urgently needed, mainly for gastrointestinal diseases and entities, of which intestinal health evaluations are currently done using macroscopic or microscopic evaluation tools, that are cumbersome in terms of costs and time. Studies have been performed to identify intestinal or faecal protein biomarkers, but due to protein stability issues in faecal material, these tools are difficult to develop in field applications. Blood biomarkers are of much higher stability, as samples can be directly analysed without interference with microbial proteases, as is the case in faecal or intestinal material.

To identify blood biomarkers, a holistic approach enabled to detect changes in the blood serum proteome, independent of the stressor. Three types of stressors of intestinal health represented by Clostridium perfringens induced necrotic enteritis, dysbiosis, and Fusarium mycotoxin deoxynivalenol (DON) were applied in experimental animal models to negatively affect the normal intestinal morphology and physiology. Within each animal experiment, a non-challenged negative control group and a challenged non-treated positive control group was included. Additionally, two challenged groups treated with a feed additive will be included. The added feed additive(s)/ingredient(s) are targeted against the corresponding stress factors. The feed additive(s)/ingredient(s) will be administered together with feed according to the manufacturer's instruction.

Mass spectrometry based proteomics has been used for the biomarkers discovery. Spectral data were recorded via Data Independent Acquisition (DIA). In recent years, novel algorithms for DIA data analysis based on machine learning such as DIA-NN became available, making it possible to interpret the complex spectra data and to benefit from the improved sensitivity and reproducibility of DIA analyses. Indeed, in DIA quantification is performed on MS2 level, leading to considerably more robust results, and helping to overtake the interfering limitations of MS1-level quantification, inherent for DDA. MS2-based quantification helps to reduce the dynamic range of within-spectra intensities.

Based on the different types of intestinal gut stressors and the spectrometry based proteomics 37 biomarkers were identified (Table 1).

**Table 1: biomarkers which are either upregulated or downregulated in gastrointestinal diseases and/or disorders in animals (infected animals) compared to healthy animals.**

| **Protein.Ids** | **Gene.names** | **Protein.names** | **Regulation in infected animals** |
|---|---|---|---|
| Q8JIG5 | ORM1 | Alpha-1-acid glycoprotein | Upregulated |
| P21611 | B2M | Beta-2-microglobulin | Upregulated |
| A0A1D5PBP6 | CP | Ceruloplasmin | Upregulated |
| A0A3Q2U7P5 | CFD | Complement factor D | Upregulated |
| A0A1L1RWP 3 | DMBT1L | Deleted in malignant brain tumors 1 protein-like | Upregulated |
| A0A1D5NW8 5 | EXFABP | Extracellular fatty acid-binding protein | Upregulated |
| F1N869 | CSF1R | Receptor protein-tyrosine kinase | Upregulated |
| F1NAP7 | MASP2 | Mannan binding lectin serine peptidase 2 | Upregulated |
| F1NPN5 | SPIA3 | Serpin peptidase inhibitor, clade A | Upregulated |
| F1NW65 | HPS5 | Serum amyloid A protein | Upregulated |
| A0A3Q2UGF 7 | LOC423629 | Uncharacterized LOC423629 | Upregulated |
| E1C733 | CCL26 | Chemokine | Upregulated |
| R4GGF1 | LRG1 | Leucine rich alpha-2-glycoprotein 1 | Upregulated |
| A0A3Q2UAF0 | APOA4 | Apolipoprotein A4 | Downregulate d |
| P02467 | COL1A2 | Collagen alpha-2(I) chain | Downregulate d |
| F1N869 | SPIA4 | Serpin peptidase inhibitor, clade A | Downregulate d |
| A0A1D5PXP9 | TGFBI | Transforming growth factor-beta-induced protein ig-h3 | Downregulate d |
| F1P256 | ITGBL1 | Integrin subunit beta like 1 | Downregulate d |
| A0A3Q2U321 | POSTN | Periostin | Downregulate d |
| P36377 | SPARC | Basement-membrane protein 40 | Downregulate d |
| F1NX21 | CD109 | CD109 molecule | Downregulate d |
| Q6QLR1 | GAL9 | Gallinacin-9 (Gal-9) (Beta-defensin 9) | Upregulated |
| 042220 | MSTN | Growth/differentiation factor 8 (GDF-8) (Myostatin) | Downregulate d |
| P07354 | HAPLN 1 | Hyaluronan and proteoglycan link protein 1 (Cartilage-linking protein 1) | Downregulate d |
| A0A1D5P703 | MGAM | Maltase-glucoamylase (alpha-glucosidase) | Downregulate d |
| A0A1D5PF62 | OVSTL | Ovostatin-like | Downregulate d |
| F1P2Y9 | SPINT1 | Serine peptidase inhibitor, Kunitz type 1 | Upregulated |
| P35440 | THBS2 | Thrombospondin-2 | Downregulate d |
| F1NK40 | A2ML4 | Alpha-2-macroglobulin-like 4 | N/A |
| Q6QLQ5 | CATHL1 | Cathelicidin-1 (CATH-1) (Fowlicidin-1) | N/A |
| A0A3Q2TSW 8 | NID2 | Nidogen 2 | N/A |
| P02701 | AVD | Avidin | N/A |
| E1BUA6 | VNN1 | Vanin 1 | N/A |
| A0A3Q2U5H 6 | SORT1 | Sortilin 1 | Upregulated |
| O93574 | RELN | Reelin | Upregulated |
| A0A3Q2UGB 8 | SELENOP1 | Selenoprotein P1 | Downregulate d |
| A0A3Q2U2H 4 | A0A3Q2U2H4 _ CHICK | Alpha-2-macroglobulin (results BLASTed) | Downregulate d |

Accordingly, in an aspect, the invention relates to a method for predicting, diagnosing, and/or evaluating a treatment of a gastrointestinal disease and/or disorder in an animal, preferably a monogastric animal such as a chicken, said method comprises the steps of analysing a collected biological sample from said animal for one or more biomarkers chosen from Table 1.

A "gastrointestinal (GI) disease" in animals refers to a specific pathological condition or medical disorder that affects the gastrointestinal (GI) tract of the animal. This includes any abnormality, illness, or ailment that impacts the functioning or structure of the stomach, intestines, oesophagus, and other digestive organs within the animal's body. GI diseases in animals can be caused by various factors such as infections, inflammation, parasites, dietary issues, congenital conditions, or other pathological processes within the GI tract. These diseases can result in a range of symptoms and can affect animals of different species.

Non-limiting examples of GI diseases in animals are colic in horses, canine parvovirus infection in dogs, equine gastric ulcers, feline inflammatory bowel disease (IBD), avian gastric yeast infection in birds, bovine Johne's disease in cattle, porcine epidemic diarrhoea in pigs, necrotic enteritis in poultry, feline pancreatitis, equine grass sickness, canine gastroenteritis, bovine viral diarrhoea (BVD), avian salmonellosis, canine gastric dilation-volvulus (GDV), equine colitis, coccidiosis in poultry, equine sand colic, bloat in cattle, canine gastritis, swine transmissible gastroenteritis (TGE), rabbit gastrointestinal stasis, feline infectious peritonitis (FIP), bovine blackleg, avian botulism, canine eosinophilic gastroenteritis, salmon poisoning disease in dogs, equine Potomac horse fever, porcine ileitis, feline megaoesophagus, canine haemorrhagic gastroenteritis (HGE).

A "gastrointestinal (GI) disorder" in animals is a broader term that encompasses any disruption, irregularity, or abnormality in the gastrointestinal system of the animal, regardless of whether it is a specific recognized disease. GI disorders can include a wide variety of conditions, both acute and chronic, affecting the GI tract of animals. These conditions may involve structural issues, functional problems, or other abnormalities related to the digestive system. GI disorders in animals may or may not have well-defined causes or distinct clinical symptoms, and they can affect animals of various species.

Non-limiting examples of GI disorders in animals are: gastrointestinal foreign body obstruction, malabsorption syndrome, gastroesophageal reflux, gastric dilatation-volvulus (bloat) in dogs, irritable bowel syndrome in cats, equine gastric impaction, constipation in rabbits, swine dysentery in pigs, canine megaoesophagus, gastric torsion in cattle, equine colonic impaction, rabbit gastrointestinal foreign bodies, megacolon in cats, canine exocrine pancreatic insufficiency (EPI), gastrointestinal nematode infections in livestock, feline constipation, canine lymphangiectasia, equine intussusception, canine small intestinal bacterial overgrowth (SIBO), rabbit cecal dysbiosis, cholangiohepatitis in cats, pyloric stenosis in dogs, avian coccidiosis, colonic torsion in horses, feline cholangitis, canine malabsorption, equine verminous colic, bovine abomasal displacement, avian proventricular dilatation disease (PDD), rabbit cecal impaction.

It will be clear to a person skilled in the art that, although many examples are listed in combination with a specific species, many of the diseases and/or conditions can be combined with other species as well. The examples of GI diseases and/or conditions should therefore not be seen as limited to the species they are mentioned with.

The term "treatment" refers to both therapeutic, prophylactic or preventive measures to reduce or prevent pathological conditions or disorders from developing or progressing.

The term "animal" refers to living organisms belonging to the kingdom Animalia, encompassing a wide range of multicellular organisms, including vertebrates (e.g., mammals, birds, reptiles, amphibians, fish) and invertebrates (e.g., insects, molluscs, crustaceans), that are not human beings.

Preferably, the animal is a monogastric animal. The term "monogastric animal" refers to an animal with a single-chambered stomach as its primary organ for digesting food. Monogastric animals have a simple stomach structure designed for the initial breakdown of ingested food through enzymatic and chemical processes. Unlike ruminants (such as cows and sheep), which have multi-chambered stomachs, monogastric animals rely on their single stomach to digest and absorb nutrients from food. Common examples of monogastric animals include dogs, cats, pigs, poultry (e.g., chickens, turkeys), and horses.

In an embodiment, said animal is a monogastric animal chosen from the group of pigs, avians species such as chicken or turkeys, rats, mice, horses, and rabbits. More preferably, said monogastric animal is a chicken.

In an embodiment, the one or more biomarkers are chosen from Table 1, such as 1, 2, 3, 4, 5, 6, 7, 8, 9, or 10 biomarkers chosen from Table 1. Analysing more than 1 biomarker has the advantage of increasing both the sensitivity and specificity of predicting, diagnosing and/or evaluating a treatment. In addition, gastrointestinal diseases can have diverse causes and manifestations. Some markers may be more relevant for specific subtypes or stages of the disease. A panel of at least two markers can help account for this heterogeneity. Also, including multiple markers can provide redundancy, which improves the reliability of the diagnostic or predictive test. If one marker's measurement is affected by external factors or variability, the presence of others can help validate the results. Further, when evaluating treatment effectiveness, multiple markers can track changes over time. This is particularly valuable for assessing whether a treatment is achieving its desired outcomes.

The method of the invention comprises the steps of analysing a collected biological sample from said animal for one or more biomarkers chosen from Table 1. In an embodiment, said biological sample is a body fluid sample chosen from the group consisting of blood and (blood) plasma, preferably plasma.

Previous studies have focussed on intestinal or faecal protein biomarkers. One of the issues with said faecal protein biomarker is their limited stability due to the presence of microbial proteases in faecal or intestinal material. Blood biomarkers are of much higher stability, as samples can be directly analysed without interference with microbial proteases. Blood and blood plasma are easily obtainable from animals, and are therefore the preferred biological samples to be analysed for biomarkers in the present method for predicting, diagnosing, and/or evaluating a treatment of a gastrointestinal disease and/or disorder in an animal.

The inventors surprisingly found that biomarkers of Table 1 are particularly indicative of a gastrointestinal disease and/or disorder in an animal, and are therefore useful in predicting, diagnosing, and/or evaluating a treatment of such disease and/or disorder.

### Predicting a GI disease/disorder:

Biomarkers can help predict the likelihood of animals developing GI diseases or disorders. For example, specific biomarkers or blood markers may indicate an increased risk of conditions like inflammatory bowel disease in dogs or colic in horses. Veterinarians and animal caregivers can use risk assessment models that incorporate biomarkers to make informed decisions about preventive measures, dietary changes, or early screening for at-risk animals.

### Diagnosing a GI disease/disorder:

Biomarkers are essential in the diagnostic process of GI diseases or disorders in animals. For instance, some biomarkers can help diagnose specific GI disorders in animals, such as food allergies in cats or pancreatitis in dogs.

### Evaluating a treatment of a GI disease/disorder:

Biomarkers can be used to assess the effectiveness of treatments for GI diseases in animals. Monitoring biomarkers levels of biomarkers indicative for said GI diseases/conditions can help veterinarians gauge the response to treatment in animals with GI conditions, allowing for adjustments in the treatment plan, and ensuring personalized and effective treatment strategies for GI conditions.

In particular, using a broiler gut inflammation paradigm of necrotic enteritis, the following blood plasma biomarkers for intestinal health of the chicken were identified (see examples 1 and 2): Table 2. Table 2 is a subsection of Table 1.

**Table 2: Biomarkers which are either upregulated or downregulated in animals suffering from necrotic enteritis (infected animals) compared to healthy animals.**

| **Protein.Ids** | **Gene.name s** | **Protein.names** | **Regulation in infected animals** |
|---|---|---|---|
| Q8JIG5 | ORM1 | Alpha-1-acid glycoprotein | Upregulated |
| P21611 | B2M | Beta-2-microglobulin | Upregulated |
| A0A1D5PBP6 | CP | Ceruloplasmin | Upregulated |
| A0A3Q2U7P5 | CFD | Complement factor D | Upregulated |
| A0A1L1RWP3 | DMBT1L | Deleted in malignant brain tumors 1 protein-like | Upregulated |
| A0A1D5NW8 5 | EXFABP | Extracellular fatty acid-binding protein | Upregulated |
| F1N869 | CSF1R | Receptor protein-tyrosine kinase | Upregulated |
| F1NAP7 | MASP2 | Mannan binding lectin serine peptidase 2 | Upregulated |
| F1NPN5 | SPIA3 | Serpin peptidase inhibitor, clade A | Upregulated |
| F1NW65 | HPS5 | Serum amyloid A protein | Upregulated |
| A0A3Q2UGF7 | LOC423629 | Uncharacterized LOC423629 | Upregulated |
| E1C733 | CCL26 | Chemokine | Upregulated |
| R4GGF1 | LRG1 | Leucine rich alpha-2-glycoprotein 1 | Upregulated |
| A0A3Q2UAF0 | APOA4 | Apolipoprotein A4 | Downregulate d |
| P02467 | COL1A2 | Collagen alpha-2(I) chain | Downregulate d |
| F1N869 | SPIA4 | Serpin peptidase inhibitor, clade A | Downregulate d |
| A0A1D5PXP9 | TGFBI | Transforming growth factor-beta-induced protein ig-h3 | Downregulate d |
| F1P256 | ITGBL1 | Integrin subunit beta like 1 | Downregulate d |
| A0A3Q2U321 | POSTN | Periostin | Downregulate d |
| F1NK40 | A2ML4 | Alpha-2-macroglobulin-like 4 | N/A |
| Q6QLQ5 | CATHL1 | Cathelicidin-1 (CATH-1) (Fowlicidin-1) | N/A |
| A0A3Q2TSW 8 | NID2 | Nidogen 2 | N/A |
| P02701 | AVD | Avidin | N/A |
| E1BUA6 | VNN1 | Vanin 1 | N/A |

In particular, using a dysbiosis model, the following blood plasma biomarkers for intestinal health of the chicken were identified (see example 3): Table 3. Table 3 is a subsection of Table 1.

"Dysbiosis" (also called dysbacteriosis) is characterized by a disruption of the microbiome in a subject resulting in an imbalance in the microbiota, changes in their functional composition and metabolic activities, or a shift in their local distribution. Dysbiosis is most commonly reported as a condition in the gastrointestinal tract, and commonly results in an increased intestinal permeability, which can be detected using mass spectrometry such as by the use of a non-invasive, nontoxic marker such as iohexol.

**Table 3: Biomarkers which are either upregulated or downregulated in animals suffering from dysbiosis (infected animals) compared to healthy animals.**

| **Protein.Ids** | **Gene.name s** | **Protein.names** | **Regulation in infected animals** |
|---|---|---|---|
| F1NAP7 | MASP2 | Mannan binding lectin serine peptidase 2 | Upregulated |
| E1C733 | CCL26 | Chemokine | Upregulated |
| R4GGF1 | LRG1 | Leucine rich alpha-2-glycoprotein 1 | Upregulated |
| A0A1D5PXP 9 | TGFBI | Transforming growth factor-beta-induced protein ig-h3 | Downregulated |
| F1P256 | ITGBL1 | Integrin subunit beta like 1 | Downregulated |
| A0A3Q2U32 1 | POSTN | Periostin | Downregulated |
| P36377 | SPARC | Basement-membrane protein 40 | Downregulated |
| F1NX21 | CD109 | CD109 molecule | Downregulated |
| Q6QLR1 | GAL9 | Gallinacin-9 (Gal-9) (Beta-defensin 9) | Upregulated |
| O42220 | MSTN | Growth/differentiation factor 8 (GDF-8) (Myostatin) | Downregulated |
| P07354 | HAPLN 1 | Hyaluronan and proteoglycan link protein 1 (Cartilage-linking protein 1) | Downregulated |
| A0A1D5P70 3 | MGAM | Maltase-glucoamylase (alpha-glucosidase) | Downregulated |
| A0A1D5PF62 | OVSTL | Ovostatin-like | Downregulated |
| F1P2Y9 | SPINT1 | Serine peptidase inhibitor, Kunitz type 1 | Upregulated |
| P35440 | THBS2 | Thrombospondin-2 | Downregulated |

In addition, by challenging chicken with the mycotoxin deoxynivalenol (DON), the following blood plasma biomarkers for intestinal health of the chicken were identified (see example 4): Table 4. Table 4 is a subsection of Table 1.

DON is a mycotoxin produced by certain moulds, particularly species of Fusarium. It is primarily known for its toxic effects on humans and animals when ingested through contaminated food or feed. DON is considered a harmful substance, and its consumption can lead to a range of health issues, including gastrointestinal disturbances, vomiting, diarrhoea, reduced feed intake, and immune system suppression.

**Table 4: Biomarkers which are either upregulated or downregulated in animals challenged with the mycotoxin deoxynivalenol (DON) (infected animals) compared to healthy animals.**

| **Protein.Ids** | **Gene.names** | **Protein.names** | **Regulation in infected animals** |
|---|---|---|---|
| A0A3Q2U5H 6 | SORT1 | Sortilin 1 | Upregulated |
| O93574 | RELN | Reelin | Upregulated |
| A0A3Q2UGB 8 | SELENOP1 | Selenoprotein P1 | Downregulated |
| A0A3Q2U2H 4 | A0A3Q2U2H4_CHIC K | Alpha-2-macroglobulin (results BLASTed) | Downregulated |

In an embodiment, the method comprises that individual levels of said one or more biomarkers of Table 1 are quantified. This can be done through any method known by the skilled person, such as through immunoassays, mass spectrometry, protein microarrays, NMR Spectroscopy, Western Blotting, protein quantification kits, or liquid chromatography.

Immunoassays are commonly used to measure the concentration of specific proteins or peptides. Enzyme-linked immunosorbent assays (ELISA) and enzyme-linked immunosorbent spot assays (ELISpot) are examples of immunoassays that can be used to quantify biomarker levels. In these assays, antibodies specific to the biomarkers of interest are used to detect and measure their concentration. Further, mass spectrometry is a highly sensitive technique that can be used to identify and quantify proteins and peptides. Liquid chromatography-mass spectrometry (LC-MS) and matrix-assisted laser desorption/ionization (MALDI-TOF) are common mass spectrometry methods used in biomarker quantification. Further, protein microarrays can simultaneously measure the levels of multiple proteins or peptides in a single experiment. This can be useful for profiling biomarker expression patterns. Further, nuclear magnetic resonance (NMR) spectroscopy can be employed to study the structure and concentration of biomolecules, including certain metabolites and proteins. Also, Western blotting is a technique used to detect and quantify specific proteins in a biological sample. It is especially useful when precise quantification of a single protein is required. Moreover, (commercially available) protein quantification kits are often used for rapid and precise measurement of protein concentrations. Also High-performance liquid chromatography (HPLC) and ultra-high-performance liquid chromatography (UHPLC) can be used for protein quantification in complex samples.

In an embodiment, said prediction, diagnosis or evaluation occurs on the basis of individual levels of said one or more biomarkers. By comparing individual levels of said biomarkers, one can easily see changes in biomarker levels which are indicative of gastrointestinal diseases and/or disorders. Comparing biomarker levels over multiple biomarkers, i.e. not on the basis of individual levels, can result in changes in one biomarker levels (e.g. upregulation) being masked by another biomarker (i.e. downregulation), because of which said gastrointestinal diseases and/or disorders could go unnoticed.

In a preferred embodiment, said quantified biomarker levels are compared to control levels of healthy animals. In a further embodiment, said levels can be higher or lower, i.e. upregulated or downregulated, compared to the control levels of healthy animals, preferably at least 1, 2, 3, 4, 5, 6, 7, 8, 9, 10, 11, 12, 13, 14, 15, 20, 25, 30, 35, 40, 45, 50, 60, 70, 80, 90, 100, 120, 150, 175, 200 % higher or lower.

Said "healthy animals" are animals that are free from any significant signs, symptoms, or clinical manifestations of gastrointestinal diseases and disorders, exhibiting animal-typical behaviours, having normal physiological functions, and show no evidence of abnormality or illness related to the gastrointestinal system.

Most preferably, the biomarkers are chosen from the upregulated biomarkers as shown in Table 1, more particularly ORM1, B2M, CP, CFD, DMBT1L, EXFABP, CSF1R, MASP2, SPIA3, HPS5, LOC423629, CCL26, LRG1, GAL9, SPINT1, SORT1, and RELN; even more preferably chosen from MASP2, CDF, and HPS5. A person skilled in the art will understand that the preference of upregulated biomarkers over downregulated biomarkers lays in the fact that biomarkers which are upregulated are easier to measure, while downregulated biomarkers are more difficult to measure. Downregulated biomarkers, although less easy to measure, are however at least as indicative of such gastrointestinal disease and/or disorder as upregulated biomarkers.

In an embodiment, said biomarkers are protein and/or peptide markers. Preferably, protein and/or peptide expression levels are measured.

In an embodiment, said analysing a collected biological sample from said animal for one or more biomarkers occurs by means of an immunoassay. Examples of such immunoassay are, as already partly described above, ELISA, Fluorescence immunoassays, including fluorescence polarization immunoassay (FPIA) and time-resolved fluorescence immunoassay (TRFIA) Chemiluminescent Immunoassay (CLIA), Enzyme Multiplied Immunoassay Technique (EMIT), Radioimmunoassay (RIA) (less commonly used today due to safety concerns associated with radioisotopes), Particle-Based Immunoassays such as latex agglutination assays and magnetic particle-based assays, Surface Plasmon Resonance (SPR), Bead-Based Immunoassays such as multiplexed assays, and finally Lateral flow assays which are however typically designed for qualitative or semi-quantitative measurements but can be adapted to detect changes in protein levels in a sample, such as in blood plasma.

In an embodiment, the gastrointestinal disease and/or disorder is caused by a bacterial, fungal, viral, or parasitic infection.

Non-limiting examples of bacterial infections or conditions include salmonella, which commonly affects various animal species, including poultry, cattle, and reptiles; Escherichia coli (E. coli) which can cause gastrointestinal infections in livestock and other animals; Clostridium perfringens which is known to cause necrotic enteritis in poultry and enterotoxemia in ruminants; and Campylobacter which can affect poultry and other animals, leading to infections and sometimes enteritis.

Non-limiting examples of fungal infections or conditions include Candida which may impact animals, especially those with weakened immune systems, such as birds or reptile; Aspergillus which can affect animals, particularly birds, causing aspergillosis in the respiratory and digestive systems; and Histoplasma capsulatum causing histoplasmosis, which may involve the gastrointestinal tract in some cases.

Non-limiting examples of viral infections or conditions include Norovirus and Rotavirus, the latter which affects mainly young animals, including calves and piglets, causing diarrhoea.

Non-limiting examples of parasitic infections or conditions include protozoan infections or conditions, helminth infections or conditions, and ectoparasitic infections or conditions. Protozoan infections or conditions include amongst others Eimeria species which can infect a variety of animals, including cattle, sheep, goats, rabbits, and various other mammals and birds. Emeria is known to cause coccidiosis, affecting the intestinal tract of animals. In particular invasion and reproduction of the protozoa cause damage to the intestinal cells, which can result in symptoms such as diarrhoea, weight loss, reduced growth rates, and, in severe cases, mortality; Giardia lamblia which can infect various animal species, including dogs and livestock; Entamoeba histolytica which may affect animals, leading to amoebic infections; Cryptosporidium which can cause cryptosporidiosis in for instance calves and lambs; Toxoplasma gondii can cause Toxoplasma mainly carried by wildlife and domesticated animals, particularly cats. Helminth (worm) infections or conditions include amongst others Ascaris lumbricoides such as in pigs or in other animals; Trichuris trichiura which may cause trichuriasis; Hookworms; and Tapeworms. Ectoparasites infections or conditions include amongst others Sarcoptes scabiei (mite) causing sarcoptic mange; and Pediculus humanus (lice).

In an embodiment, the gastrointestinal disease and/or disorder is caused by a dietary factor or environmental condition. Non-limiting examples of dietary factors include feed contaminated with (myco)toxins; water contaminated with pathogens, toxins, or heavy metals; antinutritional factors; aflatoxins produced by moulds like Aspergillus flavus and A. parasiticus; allergenic ingredients; rapid diet changes without proper transition; and nutrient imbalance i.e. lacking essential nutrients or imbalanced in key components. Non-limiting examples of environmental conditions include high temperatures and humidity levels causing heat stress for the animal; cold stress; excessive moisture resulting in wet and muddy conditions; overcrowding; stress during transportation; air pollution and related inadequate ventilation; noise pollution; and inadequate biosecurity and hygiene management resulting in the introduction and spread of diseases.

In an embodiment, the gastrointestinal disease and/or disorder is caused by Clostridium perfringens, dysbiosis, or mycotoxins.

In an embodiment, the gastrointestinal disease and/or disorder is necrotic enteritis.

Necrotic enteritis is a severe and often fatal gastrointestinal disease primarily affecting poultry, including chickens. It is caused by specific strains of the bacterium Clostridium perfringens, particularly Clostridium perfringens type A. This bacterium is commonly found in the environment and can also be a part of the normal gut microbiota in birds. Necrotic enteritis typically manifests as an acute or chronic infection of the chicken's small intestine. The disease is characterized by the following key features: gut lesions, toxin production by C. perfringens (alpha toxin: phospholipase C), severe diarrhoea, reduced feed intake, weight loss, and lethargy, high mortality rates (mortality may occur within a few days of infection). Several factors can predispose chickens to necrotic enteritis, including dietary factors (e.g., high-protein diets), coccidial infections (another type of gut infection), stress, and poor hygiene or management practices.

In a second aspect, the present invention relates to a method for evaluating the efficacy of a compound to prevent or treat a gastrointestinal disease and/or disorder in an animal, said method comprises administering to an animal an amount of said compound, and analysing one or more biomarkers chosen from Table 1.

A person of ordinary skill in the art will appreciate that elements of the aspect of the method as described above return in the second aspect relating to a method of the invention, and to further aspects of the invention relating to a biomarker panel, to an immunoassay, and to a use of a biomarker panel. Consequently, all aspects of the present invention are related. All features as described in one of the aspects, as described above as well as below, can relate to any of these aspects, even if they are described in conjunction with a specific aspect. Each aspect or embodiment so defined may be combined with any other aspect(s) or embodiment(s) unless clearly indicated to the contrary. In particular, any feature indicated as being preferred or advantageous may be combined with any other feature or features indicated as being preferred or advantageous.

In an embodiment, said method for evaluating the efficacy of a compound to prevent or treat a gastrointestinal disease and/or disorder in an animal comprises analysing said one or more biomarkers before administering said compound and after administering said compound. Preferably, said analysing of the one or more biomarkers before and after administering said compound includes quantifying said biomarkers, preferably on the basis of individual levels of said one or more biomarkers. In an embodiment, said biomarkers are obtained from a biological sample of the animal, such as blood or blood plasma.

By monitoring biomarkers levels of biomarkers indicative for GI diseases/conditions can help veterinarians gauge the response to treatment using the compound in animals with GI disease or condition, or at risk of developing said GI disease or condition. This evaluating further allows for adjustments in the treatment plan (e.g. higher/lower dose of the compound, or advising the use of a different compound) and ensuring personalized and effective treatment strategies for GI diseases/conditions.

In an embodiment, this method also relates to predicting, diagnosing, and/or evaluating the efficacy of in intervention or remedy of a gastrointestinal disease and/or disorder in an animal.

In an aspect, the invention relates to a biomarker panel for predicting, diagnosing, and/or evaluating a treatment of a gastrointestinal disease and/or disorder in an animal, preferably a monogastric animal such as a chicken, wherein said biomarker panel comprises at least two markers chosen from Table 1.

Measuring at least two makers chosen from Table 1 increases both the sensitivity and specificity of predicting, diagnosing and/or evaluating a treatment. In addition, gastrointestinal diseases can have diverse causes and manifestations. Some markers may be more relevant for specific subtypes or stages of the disease. A panel of at least two markers can help account for this heterogeneity. Also, including multiple markers can provide redundancy, which improves the reliability of the diagnostic or predictive test. If one marker's measurement is affected by external factors or variability, the presence of others can help validate the results. Further, when evaluating treatment effectiveness, multiple markers can track changes over time. This is particularly valuable for assessing whether a treatment is achieving its desired outcomes.

In an embodiment, the panel comprises at least 3, at least 4, or at least 5 markers chosen from Table 1.

In an aspect, the invention relates to an immunoassay for predicting, diagnosing, and/or evaluating a treatment of a gastrointestinal disease and/or disorder in an animal, wherein said immunoassay analyses at least two markers chosen from Table 1.

In an aspect, the invention relates to a use of a biomarker panel for predicting, diagnosing, and/or evaluating a treatment of a gastrointestinal disease and/or disorder in an animal, preferably a monogastric animal such as a chicken, wherein said biomarker panel comprises one or more biomarkers as listed in Table 1.

The present invention will be now described in more details, referring to examples that are not limitative.

### EXAMPLES

Note: the below examples focus on experiments performed on and/or with poultry, pigs, or both. However, as shown in Example 1 below for chicken and human (90%), is a high similarity of blood plasma proteome to be expected in other monogastric animals as well. Biomarkers obtained in the below examples are thus very likely to be indicative of gastrointestinal disease/disorder in other monogastric animals, such as but not limited to chicken, pigs, dogs, cats, rats, mice, rabbits, and horses.

### Example 1: necrotic enteritis

The aim of the present study was to identify biomarkers related to intestinal health and disease/disorders in animals, the present example focussing on chicken suffering from necrotic enteritis. For this, data dependent acquisition (DDA) and data independent acquisition (DIA) methods were used and compared.

### 1. Materials and Methods

### 1.1 Experimental design: in vivo necrotic enteritis model

In the current study the NE model previously described by Gholamiandehkordi et.al (2007) was used, with some modifications. Briefly, one-day-old non-vaccinated Ross 308 broiler chicks were purchased from a local hatchery and randomly allocated into challenged and non-challenged groups with 80 and 20 animals in each. Birds were provided with ad libitum access to feed and water. Until day 17 of the experiment all birds received a wheat/rye-based (43%/7.5%) diet with soybean meal as protein source (30%). Afterwards the source of protein in the diet was replaced with fishmeal (30%). On days 14 and 16 birds from the challenge group where orally treated with 10-fold dose of live attenuated Eimeria vaccine Paracox-5 (containing E. Acervulina HP, E. maxima CP, E. maxima MFP, E. mitis HP, E. tenella HP) (MSD Animal Health, Brussels, Belgium). This type of feed is characterized by high levels of non-starch polysaccharides that lead to digestibility losses, and high levels of poorly digestible proteins. This diet composition predisposes broilers to the development of NE. The netB positive C. perfringens type G strain CP56 was cultured in BHI broth (Sigma Aldrich, St. Louis, Missouri, US) overnight at 37°C under anaerobic conditions. Consecutively, broilers from the treatment group received an oral dose of approximately 5 × 10⁸ CFU of pathogenic C. perfringens strain CP56 on days 18 and 19. All animals were euthanized on day 20 of the experiment. Prior to euthanasia, 3ml blood was withdrawn and collected into EDTA-treated vacutainers for further separation of plasma (1900rcm during 10 min at room temperature, with no brake in the swinging bucket centrifuge). Vacutainers with blood were inverted five times prior to centrifugation; after, a portion of plasma was carefully pipetted into a new tube, preventing contact between the pipette tip and 'buffy coat'-part of the extract thus collection of the bottom 2-5 mm layer of plasma was omitted. Each sample of plasma was aliquoted, snap frozen in liquid nitrogen and stored at - 80°C for further analysis. For evaluating severity of NE lesions, the macroscopic scoring of small mid-intestinal segments (duodenum, jejunum, ileum) of all birds was performed according to the scoring system established by Keyburn et al. (2006). Birds with a lesion score of 2 and higher were classified as NE-positive.

All the experiments and manipulations involving animals were approved by the ethical committee of the Faculties of Veterinary Medicine and Bioscience Engineering of Ghent University (EC2020-045).

### 1.2 Proteomics sample preparation

The proteomics analysis was performed on plasma samples obtained from birds with the most severe NE lesion score (score 5-6, n= 17) and birds that received no treatment (score 0, n=9). Sample sizes were determined based on research on faecal protein biomarker and using G*Power software (ver. 3.1.9.2, Kiel, Germany) with applied parameters of o= 0.05 and power= 95%. Of each sample 1 µl plasma was added to 50 µl S-trap buffer containing 5% sodium dodecyl sulfate (SDS) and 50 mM triethylammonium bicarbonate (TEAB), pH 7.55. Proteins were reduced by addition of 15 mM dithiothreitol and incubation for 30 minutes at 55°C and then alkylated by addition of 30 mM iodoacetamide and incubation for 15 minutes at RT in the dark. Phosphoric acid was added to a final concentration of 1.2% and subsequently samples were diluted 7-fold with binding buffer containing 90% methanol in 100 mM TEAB, pH 7.1. The samples were loaded on the 96-well S-Trap^{™} plate (Protifi), placed on top of a deepwell plate, and centrifuged for 2 min at 1,500 x g at RT. After protein binding, the S-trap^{™} plate was washed three times by adding 200 µl binding buffer and centrifugation for 2 min at 1,500 x g at RT. A new deepwell receiver plate was placed below the 96-well S-Trap^{™} plate and 50 mM TEAB containing trypsin (1/100, w/w) was added for digestion overnight at 37°C. By centrifugation for 2 min at 1,500 x g, peptides were eluted in three times, first with 80 µl 50 mM TEAB, then with 80 µl 0.2% formic acid (FA) in water and finally with 80 µl 0.2% FA in water/acetonitrile (ACN) (50/50, v/v). Eluted peptides were dried completely by vacuum centrifugation and stored at -20°C until further use.

### 1.3 LC-MS/MS analysis

For DDA analysis, purified peptides of all 26 samples were re-dissolved in 20 µl loading solvent A (0.1% TFA in water/ACN (98:2, v/v) and the peptide concentration was determined on a Lunatic spectrophotometer (Unchained Labs). From each sample 5 µl was injected for LC-MS/MS analysis on an Ultimate 3000 RSLCnano system in-line connected to a Q Exactive HF mass spectrometer equipped with a Nanospray Flex^{™} Ion Source (Thermo Fisher Scientific). Trapping was performed at 10 µl/min for 4 min in loading solvent A on a 20 mm trapping column (made in-house, 100 µm internal diameter (I.D.), 5 µm beads, C18 Reprosil-HD, Dr. Maisch, Germany). The peptides were separated on a 250 mm nanoEase column (M/Z HSS T3 column, 100 Å, 1.8 µm, 75 µm I.D., Waters) kept at a constant of 45°C. Peptides were eluted by a non-linear increase from 5 to 55% MS solvent B (0.1% FA in water/ACN (2:8, v/v)) over 145 minutes, at a flow rate of 300 nl/min, followed by a 10-minutes wash reaching 99% MS solvent B and re-equilibration with 95% MS solvent A (0.1% FA in water). The mass spectrometer was operated in data-dependent mode, automatically switching between MS and MS/MS acquisition for the 12 most abundant ion peaks per MS spectrum. Full-scan MS spectra (375-1,500 m/z) were acquired at a resolution of 60,000 at 200 m/z in the Orbitrap analyser after accumulation to a target value of 3,000,000. The 12 most intense ions above a threshold value of 13,000 (minimum AGC of 1,000) were isolated for fragmentation at a normalized collision energy of 28%. The C-trap was filled at a target value of 100,000 for maximum 80 ms and the MS/MS spectra (200-2,000 m/z) were acquired at a resolution of 15,000 at 200 m/z in the Orbitrap analyser with a fixed first mass of 145 m/z. Only peptides with charge states ranging from +2 to +6 were included for fragmentation and the dynamic exclusion was set to 20 s. QCloud was used to control instrument longitudinal performance during the project.

For DIA analysis, from each of the 26 samples redissolved in loading solvent A as described above, the peptide concentration was adjusted to 0.015 µg/µl with 0.1% FA. iRT peptides (Biognosys, PIN Ki-3002-1) were added to each sample according to the manufacturer's instructions. Then, 300 ng of each sample was loaded on an Evotip (Evosep, PIN EV2003) according to the manufacturer's instructions. All loaded Evotips were stored in 0.1% FA at 4°C until LC-MS/MS analysis could be started. Samples were run in data-independent mode on an Evosep One LC-system (Evosep, Denmark) in-line connected to a Q Exactive HF mass spectrometer (Thermo Fisher Scientific). Peptides were analyzed with the 15 SPD method (88 min run length) using the endurance Evosep column (15cm × 150 µm I.D., 1.9µm beads, Evosep, Denmark). For elution of the peptides from the column solvent A consisted of 0.1% FA in LC-MS-grade water and solvent B consisted of 0.1% FA in ACN. Full-scan MS spectra ranging from 375-1500 m/z with an AGC target value of 5E6, a maximum fill time of 50 ms and a resolution of 60,000 at 200 m/z were followed by 30 quadrupole isolations with a precursor isolation width of 10 m/z for HCD fragmentation at an NCE of 30% after filling the trap at a target value of 3E6 for maximum injection time of 45 ms. MS2 spectra were acquired at a resolution of 15,000 at 200 m/z in the Orbitrap analyser without multiplexing. The isolation intervals ranging from 400 - 900 m/z were created with the Skyline software tool. QCloud was used to control instrument longitudinal performance during the project.

### 1.4 Proteomics data analysis

Raw files corresponding to the 26 DDA runs were searched together using the MaxQuant software (version 2.0.3.0) with mainly default search settings, including a false discovery rate set at 1% on peptide and protein level. Recorded spectra were searched against the Gallus gallus proteins present in the UniProt database (reference proteome UP000000539 containing 18,112 sequences, downloaded from www.uniprot.org, release from June 2022). The mass tolerance for precursor and fragment ions was set to 4.5 and 20 ppm, respectively, during the main search. Enzyme specificity was set as C-terminal to arginine and lysine, also allowing cleavage at proline bonds with a maximum of two missed cleavages. Variable modifications were set to oxidation of methionine residues and acetylation of protein N-termini whereas carbamidomethylation of cysteine residues was set as fixed modification. Matching between runs was enabled with a matching time window of 0.7 minutes and an alignment time window of 20 minutes. Only proteins with at least one unique or razor peptide were retained. Proteins were quantified by the MaxLFQ algorithm integrated in the MaxQuant software. A minimum ratio count of two unique or razor peptides was required for quantification.

Raw files corresponding to the 26 DIA runs were searched together using the DIA-NN software (version 1.8.1) with mainly default settings and in combination with a library-free search with selected FASTA digest using the same protein database as described for DDA. Trypsin was selected as the digestion enzyme (allowing cleavage N-terminally at proline and allowing up to two missed cleavages), and carbamidomethylation of cysteines, oxidation of methionines and acetylation of N-termini were set as variable modifications, with maximum number of variable modifications set to five. The maximum number of trypsin-missed cleavages was set to two. Precursor mass range filter was set at 400-900 m/z, the match between runs (MBR) option which was enabled, and "mass accuracy" (MS2 mass accuracy) was set at 20 ppm and "MS1 accuracy" (MS1 mass accuracy) at 10 ppm.

Further statistical analysis was performed using the RStudio software (version 4.1.1). Peptide expression matrices were prepared as follows: for DDA, the MaxQuant peptides output table was used after removal of reverse database hits; for DIA, the DIA-NN main report output table was filtered at a precursor and protein library q-value cut-off of 1 % and only proteins with at least one proteotypic peptide were retained. Protein expression matrices were prepared as follows: for DDA, the MaxQuant proteinGroups output table was used after removal of reverse database hits and hits only identified by site; for DIA, the DIA-NN main report output table was filtered at a precursor and protein library q-value cut-off of 1 % and only proteins with at least one proteotypic peptide were retained. Reported peptide and protein ID rates took into account features detected in at least two samples. For differential abundance analysis, MaxLFQ-normalized protein intensities were log2 transformed and proteins with less than four valid values in at least one experimental condition were removed, leading to a list of 295 and of 344 quantified proteins in the experiment for DDA and DIA, respectively. Missing values were imputed by random sampling from a normal distribution centred around the noise level (package DEP). Pairwise comparison testing between necrotic enteritis (NE) and control (Ctrl) groups was then carried out using the limma package, with statistical significance for differential regulation set at an FDR cut-off value of 0.05 and a |log2(fold change)| above 1.

Gallus gallus to human orthology search was done using the HCOP tool from the HUGO Gene Nomenclature Committee, allowing to extract 295 human orthologs from a list of 334 chicken plasma proteins. Protein relative abundances were calculated using the get_iBAQ function from the package DIAgui, using default settings. Plasma protein functional categories were retrieved from Anderson and Anderson (2002) and human plasma MS-based proteomics data came from the Human Plasma PeptideAtlas (2021-07 build).

### 1.5 Gene ontology (GO) analysis of differentially expressed genes

For regulated proteins, discovered upon statistical testing for differences between the two-group means, the gene ontology analysis was performed employing g:Profiler tool (https://biit.cs.ut.ee/gprofiler/gost). Regulated proteins discovered with DDA and DIA were used for query separately. The IDs of the proteins of interest were used as input data and Gallus gallus was chosen as the query organism. The g:GOSt function in the input panel was selected to performs functional enrichment analysis and highlight the driver terms in GO. The g:GOSt applies Fisher's one-tailed test to estimate the difference the query and the ontology term. A threshold for p-value was set to 0.05, same as a default option of the g:Profiler for additional filtering of the results. All data sources available in the tool were enabled for searches, in addition to Gene Ontology (http://geneontology.org/). These included human phenotypes from Human Phenotype Ontology, pathways from KEGG Reactome (https://reactome.org/) and WikiPathways (https://www.wikipathways.org/), miRNA targets from miRTarBase (https://mirtarbase.cuhk.edu.cn/), and regulatory motif matches from TRANSFAC (http://genexplain.com/transfac/). In order to categorize proteins, GO functional annotations for biological processes, molecular functions, and cellular components were used. Obtained categorized results were on a form of bubble plot and presented in the form of bubble maps, based on the adjusted p-value (x-axis).The size of the bubble corresponds to the number of genes assigned to the particular GO-term.

### 1.6 Measurements of concentrations for regulated protein by immunoassay ELISA

To evaluate how the analytical performance of MS compares to that of immunoassays and to test the potential candidate biomarkers for chicken gut health in plasma samples of infected and non-infected birds, three commercially available ELISA kits were performed. The immunoassay kit for serum amyloid protein (HPS5), complement factor D (CFD) and Mannan Associated Serine Protease 2 (MASP2) were obtained from Abbexa LTD (abx257741,Abbexa LTD, Cambridge, UK) and MyBioSource (CFD, MBS2503729; MASP2, MBS2506173, MyBioSource, San Diego, CA, USA), respectively. The assays were executed according to the instructions of manufacturers, applying dilution factor of 1:1000 for HPS5, 1:7 for CFD, and 1:2 for MASP2, testing each individual sample in duplicate.

Statistical analysis on immunoassays was performed in GraphPad Prism (version 8.4.3) software by non-parametric testing considering the size of each group and distribution. Differences between NE and Ctrl groups were assessed by Mann-Whitney test; correlation between these proteins, was assessed by a Spearman rank test. Correlation coefficients between the two MS-methods and immunoassays were analysed by Spearman rank test.

### 2. Results

Particularly during haemolysis, apoptosis, and necrosis, the content of cells - reservoirs rich in protein, are liberated and dissolved in the blood plasma. The ability to detect these proteins in blood samples is a supreme advantage of using proteomics to identify disease biomarkers. These proteins can already be considered as test targets for the clinical pathology, thus as biomarkers. In order to thoroughly examine the alterations to the blood proteome upon severe gut inflammation using strong and sensitive proteomics methods, necrotic enteritis, an enteric illness, was administered to the chickens in this work.

### 2.1 Challenged birds develop severe necrotic enteritis

We assume that NE is the most suitable in vivo model that ensures significant impactful intestinal modifications, and allows to explore the impairment of the gut functioning on the blood plasma level. Due to impact of this disease on the gut health of broilers, the in vivo model of NE was applied in the present study.

During the in vivo experiment, two birds from the challenge group died prior to euthanasia and were excluded from further analysis. Each animal was given an NE lesion score after macroscopic observation of three segments of the small intestine. The NE lesion score was calculated as the average of the scores estimated for the middle parts of small intestinal segments.

The results showed that the majority of birds developed the clinical form of the disease, i.e., severe necrotic enteritis (lesion scores 5 and 6 corresponding to 29.5% and 24.4% of all birds, respectively), while an equally high number of animals suffered from a subclinical NE state (lesion scores 3 and 4, i.e., 9% and 28.2%, respectively). The tendency towards severe NE was most likely induced by the type of Eimeria species present in the vaccine, which was administered to induce coccidiosis, the predisposing infectious factor of NE.

In this study, animals received a 10-fold dose of Paracox-5 vaccine twice (containing E. acervulina, E. maxima CP, E. maxima MFP, E. mitis, and E. tenella), unlike other studies that alternated with Hipracox (containing E. tenella, E. acervulina, E. maxima, E. praecox, and E. mitis). Introducing certain species in repetition may have had a significant impact on the gut health of birds, resulting in a higher incidence of NE in the clinical form. The results are presented in more detail on Figure 1; percentage values describe a fraction of the total number of birds challenged with C. perfringens with assigned lesion score.

### 2.2 DIA analysis leads to more complete plasma proteomics data in chicken

Plasma samples from broiler chickens with the most severe form of NE (i.e. le¬sion score 5-6, n=17) and from healthy birds (non-challenged, n=9) were selected for proteomics LC-MS/MS analysis and processed by both standard data-dependent (DDA) and emerging data-independent (DIA) acquisition modes; the recorded spectral data was analyzed by the MaxQuant and DIA-NN software, respectively. Besides the difference in spectral acquisition and data analysis, also the LC conditions differed between both workflows. While in the DDA workflow peptides were analyzed with 3-hour LC-MS/MS runs, for DIA faster 1.5-hour runs were used.

To evaluate the performance of both workflows we first looked at the number of identified peptides and proteins. In total, the DDA and DIA workflows led to the identification of 576 and 447 proteins, respectively, however, after filtering to retain proteins that were found in at least 2 samples these numbers reduced to 318 and 362 (Figure 2A). Almost half of these proteins (46.6%) were shared between both workflows, supporting their high confidence identification (Figure 2B). In total, we identified 7469 and 5221 peptides for the DDA and the DIA workflow, respectively (Figure 2C). Again, roughly half of these sequences (52.9%) was shared between both workflows (Figure 2D). Interestingly, both approaches resulted in higher numbers of identified peptides and proteins in the NE- samples compared to the control samples, hinting towards a potential difference in protein content between both biological conditions.

Secondly, we evaluated the completeness of the recorded datasets. We observed that the DIA data was more complete allowing to quantify 289 proteins (out of 362, or 80%) across half of the samples, versus only 219 proteins (out of 316, or 69%) in DDA (Figure 2E). Similarly, heatmap visualization of all protein intensities indicated lower numbers of missing values in DIA compared to DDA, especially for lower-intensity proteins in control samples (greyer area at the bottom, leftmost part of the heatmap) (Figure 2F). Although in both datasets the majority of missing information was found among the least abundant proteins, the dispersion of missing data was more even in DIA, reflecting the systematic sampling of peptides inherent to the method versus the stochastic selection of peptides performed in DDA mode. Taken together, despite shorter LC-MS/MS runs and lower number of initial identifications, the DIA workflow outperformed the DDA workflow in number of quantified proteins and data completeness.

### 2.3 The chicken plasma proteome covers plasma and tissue leakage proteins

With nearly 300 quantified plasma proteins, we set out to characterize the chicken blood plasma proteome in healthy individuals. A total of 334 proteins could be detected in the control group samples of the most comprehensive DIA dataset, covering roughly 6 orders of magnitude (Figure 3A). To get an idea about the similarity between chicken and human plasma, we extracted human ortholog information (295 orthologs could be recovered). Mapping to a list of 109 proteins approved as serum/plasma analytes by the FDA and with precisely attributed functional categories confirmed there are many proteins in common between the two species, with plasma intrinsic proteins (n=49) being present in a wide range of abundances, while tissue leakage proteins (n=8) appeared as expected at the lower half of the abundance curve. Of note, a very similar distribution of these protein classes was observed for the DDA dataset (Figure 3B), corroborating their high confident quantification. Finally, we compared the composition as well as the abundance levels of the chicken plasma proteins identified in this study with those of the human counterpart taking advantage of the comprehensive data from the Human Plasma PeptideAtlas build, containing over 5,000 proteins detected in 240 MS-based proteomics experiments, including quantification information. Remarkably, from the 295 chicken proteins having a human ortholog, 265 (~90 %) were present in the human plasma proteome resource and their protein abundance ranks correlated very well (Figure 3C). Thus, comparison of our dataset with the human plasma proteome supported the high confident quantification of chicken plasma and tissue leakage proteins and indicated an overall high similarity between both species.

### 2.4 Necrotic enteritis leads to significant changes in the plasma proteome

We then took a closer look at the plasma proteome changes induced by NE. To this end, we first compared the precision of quantification in both workflows using the intensity values of 175 proteins that were quantified in both DDA and DIA. Overall similar coefficients of variation (CV) were observed with values fluctuating around 50% CV for both workflows (Figure 4A). These relatively high values represent a considerable degree of biological variation between individual animals, similar to what has been reported for humans. While the % CV values obtained with both workflows were very similar to each other within the birds of control group, the opposite was observed for animals with NE. We hypothesize that high rate of variation might be induced by inter-individual difference among the samples since in our study an animal was considered as independent statistical unit in the experiment.

Therefore, considering the specificity of the disease, it would be naive to anticipate the same level of expression of particular protein within each bird. Moreover, when comparing the log2 (NE/control) fold change (FC) values for the 175 common proteins a mediocre positive correlation with a low p-value was observed (Spearman correlation test; R= 0.47, p =5.6e-11), further supporting similar and high confident quantification of plasma proteome changes induced by NE (Figure 4B).

To reveal proteins that were significantly regulated between the control and NE animals, statistical testing for differences between the two group means was performed with limma. For both workflows all quantified proteins were included (n=295 for DDA and n=344 for DIA) and statistical significance for differential regulation was set at an FDR < 0.05 and |log2FC| ≥ 1. This analysis led to 38 and 30 proteins that were significantly upregulated in the NE animals as measured by DDA and DIA, respectively, of which 11 proteins were overlapping in both workflows. Similarly, 18 and 29 proteins were significantly downregulated in the NE animals, with 4 overlapping candidates in both approaches. These 15 commonly regulated proteins are listed in Table 5, indicating the type of regulation (up- or down-) observed in the animals of the NE group. The significant regulated proteins are visualized on volcano plots of the Figure 4C, separately for DDA (Figure 4C- top) and DIA (Figure 4C - bottom).

**Table 5: Proteins discovered as overlapping between the two tested methods DDA and DIA, significantly different in abundance between control and challenged animals.**

| **Protein name** | **Accession number** | **Gene name** | **Adjusted *p-*value** | | **Regulation** |
|---|---|---|---|---|---|
| | | | ***DDA*** | ***DIA*** | |
| **Alpha-1-acid glycoprotein** | Q8JIG5 | ORM1 | 2.10E -03 | 1.00E -06 | Upregulated, ↑ |
| **Regulation in infected animalsBeta-2-microglobulin** | P21611 | B2M | 2.60E -02 | 2.80E -02 | Upregulated, ↑ |
| **Ceruloplasmin** | A0A1D5PBP6 | CP | 1.90E -07 | 1.00E -06 | Upregulated, ↑ |
| **Complement factor D** | A0A3Q2U7P5 | CFD | 8.70E -07 | 1.80E -06 | Upregulated, ↑ |
| **Deleted in malignant brain tumors 1 protein-like** | A0A1L1RWP3 | DMBT1L | 8.30E -04 | 2.30E -02 | Upregulated, ↑ |
| **Extracellular fatty acid- binding protein** | A0A1D5NW8 5 | EXFABP | 8.30E -04 | 1.20E -02 | Upregulated, ↑ |
| **Receptor protein- tyrosine kinase** | F1N869 | CSF1R | 9.10E -03 | 1.20E -02 | Upregulated, ↑ |
| **Mannan binding lectin serine peptidase 2** | F1NAP7 | MASP2 | 2.30E -06 | 2.60E -03 | Upregulated, ↑ |
| **Serpin peptidase inhibitor, clade A** | F1NPN5 | SPIA3 | 5.10E -04 | 3.70E -05 | Upregulated, ↑ |
| **Serum amyloid A protein** | F1NW65 | HPS5 | 4.00E -05 | 4.80E -06 | Upregulated, ↑ |
| **Uncharacterize d LOC423629** | A0A3Q2UGF7 | LOC42362 9 | 1.50E -02 | 4.60E -03 | Upregulated, ↑ |
| **Apolipoprotein A4** | A0A3Q2UAF0 | APOA4 | 5.60E -05 | 2.90E -03 | Downregulated , ↓ |
| **Collagen alpha- 2(I) chain** | P02467 | COL1A2 | 2.20E -02 | 1.20E -02 | Downregulated , ↓ |
| **Serpin peptidase inhibitor, clade A** | F1N869 | SPIA4 | 4.10E -06 | 7.40E -05 | Downregulated , ↓ |
| **Transforming growth factor- beta-induced protein ig-h3** | A0A1D5PXP9 | TGFBI | 2.80E -04 | 8.50E -04 | Downregulated , ↓ |

Detected significant differentially proteins per method were divided per type of regulation, observed in challenged birds (NE group), and the overlapping intersections are mentioned on Venn diagrams of the Figure 4D (left - downregulated, right - upregulated overlapping proteins).

Accordingly, having compared two workflows for plasma proteome quantification modified by induced NE allowed us to conclude that both DDA and DIA had similar coefficients of variation, evaluated for the same discovered proteins (n=175), with notably higher rate of variation observed for animals with NE. The study also identified 15 proteins that were commonly regulated between the control and NE animals. Upon statistical analysis for differences between the two groups, 56 and 59 proteins were found to be of significant differential regulation in the NE animals as measured by DDA and DIA, respectively.

### 2.5 Necrotic enteritis induces significant changes in the regulation of various categories of plasma proteins

We performed gene ontology (GO) analysis using the g:Profiler analysis tool for the lists of proteins discovered as differentially regulated between the two experimental conditions. Gene enrichment analyses were performed separately for two sets of 56 and 59 proteins of interest that were detected with DDA and DIA methods, respectively. The obtained queries filtered, thus, only the GO-terms with p-value (adjusted) less than 0.05 were kept. All the assigned GO-terms are shown in Figure 5A (top plot-DDA, bottom -DIA).

Among queried DDA-entries mapped to 19 functional GO-groups, among which 11 were related to biological processes (BP), 7 functional groups corresponded to cellular components (CC); and 1 was assigned to the category of molecular functions (MF). Most of the enriched genes were related to the functional groups of biological process, vast majority of which is involved in versatile types of immune response (GO:0006958, GO:0002455, GO:0006956, GO:0002449, GO:0002252, GO:0002460, GO:0002443, GO:0002250, GO:0016064, GO:0019724), and synapse pruning (GO:0098883). Defined enriched terms of the CC-category are mainly related to lipid- or lipid-protein complexes (GO:0034364, GO: 1990777, GO:0034358, GO:0032994) and component of collagen structures (GO:0005581). Discovered enriched term of the MF-category appears to participate in management of fatty acids (GO:0005504).

For the group of the DIA-discovered regulated proteins, in total seven genes were found enriched, among which are three terms from the BP niche, two corresponding to CC and two of MF. As for the BP category, differentially regulated proteins were vastly correlated with defence response to bacteria (GO:0050829, GO:0042742) and response to ketone (GO: 1901654), whereas in the CC category, the enriched proteins corresponded to extracellular space (GO:0005615) and extracellular region (GO:0005576) terms. The MF group was represented by glycosaminoglycan binding (GO:0005539) and heparin binding (GO:0008201) enriched proteins. Additionally, none of the queried proteins, independently from the acquisition method, was found enriched in the KEGG pathway analysis.

Based on the GO results, a predominance of differentially regulated immune functions and defence response to bacteria was observed upon the GO-analysis. These findings highlight the crucial roles of the immune system regulation, synaptic development, and lipid-related processes, along with corresponding proteins, affected by the necrotic enteritis, offering valuable insights into the underlying mechanisms of the disease.

### 2.6 The protein concentrations correlate with their expression levels

Experimental validation of the biomarker molecules is a crucial step in the process of the biomarker development. Thus, we evaluated how the results discovered with both of the tested data acquisition methods correlate with generally accepted laboratory method of protein detection - ELISA.

Significant difference among the plasma concentrations of the proteins CFD, HPS5, and MASP2 was discovered between the non-infected and infected animals. As shown on Figure 6, in vast majority of the samples from the birds which developed severe necrotic lesions (NE-group, score 5-6), significantly higher levels of all three tested proteins were detected (CFD= 5,87 ± 5,5; HPS5= 22 ± 7,1; MASP2= 24,1 ± 7,47). As opposed to the NE-group, the expressions of CFD, HPS5, and MASP2 were discovered as much lower (CFD= 0,79 ± 0,3; HPS5= 15 ± 2,3; MASP2= 3,49 ± 3,011). Furthermore, we observed a positive correlation between the expression levels of the proteins of interest. CFD manifested a high tendency to correlate with MASP2 (r = 0.62, p < 0.003), while correlation with HPS5 was found to be weaker, but positive (r = 0.31, p < 0.1). Despite minor, but positive correlation between MASP2 and HPS5 was observed (r = 0.27, p < 0.25).

In each experimental group, the concentrations of the proteins, estimated with immunoassays, reflected expression tendency, detected with DDA and DIA. Observed similarity was supported by the level of correlation estimated between the methods. Comparatively strong relation between DDA/DIA and ELISA-detected concentrations was estimated for HPS5 (r > 0.6). As well, positive correlation was found for CFD (r > 0.3 with both DDA/DIA) and MASP2 (r = 0.5 and 0.4 with DDA and DIA, respectively) proteins.

Therefore, a positive link between the expression levels of the proteins of interest, together with significant variations in the plasma concentrations of the proteins CFD, HPS5, and MASP2 between non-infected and infected animals were found. The amount of correlation obtained between the approaches corroborated the observed similarities, and the quantities of the proteins determined with immunoassays followed the expression trend discovered with DDA and DIA. The results endorse the strong agreement between the methods of protein detection, as well as the application of the omics approach for the purpose of biomarker discovery in chicken

### 3. Discussion

In broilers, balanced gastrointestinal functioning is crucial, if not the key determinant of animals performance and wellbeing. Minor-to-obvious disturbances into the physiology and structural integrity of the gut might have negative effect not only on the broilers' health, but lead to overall significant economic losses. We assume that availability of biomarkers to indicate the malignant changes to the gut health would be of significant aid to the broiler industry. Therefore, in the present work, we examined and compared the properties of two major proteomics approaches Data Dependent (DDA) and Data Independent (DIA) for data acquisition to explore the changes in chicken blood plasma induced by enteric disease of necrotic enteritis. Based on the difference between the protein regulation we defined a list of potential biomarker proteins to evaluate the gut health of broilers.

Our first objective was to compare the performance of the two approaches by analysing the total number of peptide and protein identifications. Despite high overlap between DDA and DIA on both levels (52.9% for peptide sequences, 46.6% for protein IDs), the higher rate of peptides was detected with mass spectrometer operating in DDA mode, which is a remarkable result considering the stochastic nature of data recording by this method. These findings are in good agreement with the results of similar study. Prior to applied data filtering, the same trend was found on the level of protein IDs as well: filtering to keep proteins that were present in at least two samples resulted in 318 and 362 identified proteins in DDA and DIA, respectively. Considering the difference between analysed biological samples, the ID reduction for DDA should have rather been foreseen: multiple proteins were found in a single sample only (data not shown), while, given to the time efficiency, this bias could be (almost fully) omitted in DIA thanks to comprehensively and repeated examination of every peptide in the sample digest. Another study to evaluate differences between data acquisition approaches also reported higher number of protein identifications with DDA than DIA. The authors explain that the reduction in ID count, as well as quantitative reproducibility correlated with the length of the MS run, and, thus, extension of the operating time would increase the percentage of identifications, though negligibly (5% only).

Next, we evaluated the differences in reproducibility rate between the DDA and DIA searches. Same as in previous studies, considerably higher amount of missing values was observed with DDA, which also reflected on the consistency of protein identifications between the samples. In DIA, the completeness is achieved by the method of precursor sampling - within regular timeframes, which inevitably leads to recording the data on peptide fragment ions more uniformly.

Our results show that, quantitative performance and improved reproducibility are superior with DIA, rather than DDA. These differences might be induced by multiple factors, among which is the difference in the technical setup: along with spectral data interpretation and collection differences, LC conditions varied across the two procedures. While speedier 1.5-hour runs were employed for DIA, the DDA approach required 3-hour LC-MS/MS runs to examine peptides. Secondly, the various software tools in use can help to partially explain the divergence in the output. To slightly balance the difference introduced by software suites, relaxed type of protein querying --relaxed-prot-inf, available within the entry settings of DIA-NN, was enabled. As suggested by the developers of the DIA-NN, relaxed grouping functions in the heuristic mode, allows to omit bias towards the algorithm itself and additionally is favourable to use for the benchmarking and optimization of the method. The reason to it could lie in incorporated reliance of DIA-NN on proteotypic peptides (i.e. the ones that can be quantitatively analysed by MS and represent target protein in unique manner) when performing identification and quantification. That is to say, DIA-NN suite is governed by the rule 'protein = gene product' and thus reports unique peptides, in the default settings remain unchanged.

The dynamic concentration range of proteins observed in human plasma is vast, which might pose complications once identifying the proteins of small molecular weight or low abundance. The present study explored the constitution of the chicken plasma by characterizing the distribution of protein concentrations with a reference to human proteome. Here, we compared the blood plasma proteomes of the chicken and human, using a reference list of human plasma analytes discovered rank of the proteins order in the plasma of chickens reaches roughly seven. Additionally, high level of agreement between human and chicken ortholog proteins was discovered: minimum 80 % of plasma proteins were shared between chicken and human proteomes (i.e. 278 out of the 295 having a human ortholog). The number of proteins shared by the two species was confirmed by mapping to a list of 109 proteins approved as serum/plasma analytes by the FDA and with precisely assigned functional categories. Plasma intrinsic proteins (n=49) were present in a wide range of abundances, while tissue leakage proteins (n=8) appeared as expected at the lower half. The research to sequence the chicken genome is still ongoing, hence the database's resources are currently relatively short in information. It is important to note that current analysis is limited by the quality and completeness of available proteomic data for chicken, which could substantially affect the accuracy of the results. Withal, it is foreseen the number of chicken blood plasma proteins that can be identified at high stringency will significantly rise with the annotation of the chicken genome. To the best of our knowledge, this is the only study that contributes to the understanding of the blood proteomes of chicken and human based on the comparison of orthologs.

As a follow-up checkpoint of identification reproducibility of the data analysis approaches, we evaluated the coefficients of variation (CV) of the number of proteins identified as common between DDA and DIA methods. Similarly as previously reported, in the present study we observed positive correlation between the number of readouts (protein ID's) and the variation among the samples the common proteins. The results show that for the samples of the challenged birds with higher number of identified proteins the overall CV value per group tends to be higher. We assume that, unlike for intact plasma samples i.e. ctrl group, the sparsity of the CV values within the group of challenged birds must be heavily influenced by the nature of the biological material itself submitted for analysis. These findings compare with the results of a previous study on urinary proteome: in the work it was detected that interpersonal variability contributed 47.1% to total variability and, finding no dependence of a protein variability on its abundance, the authors concluded that rather than technical aspects, these variations capture physiological aspects. The same results were reported in another study: the biological duplicates demonstrated greater variation between samples. In our study, as expected and verified upon the downstream analysis, the higher ID/precursor count was mainly originating from the samples of infected birds (NE-group). This heterogeneity occurs due to the leakage of proteins in response to the infection-induced increased gut permeability, while intact plasma retains little-to-no variations independently from method of analysis. Coupling the fact that each animal was considered as statistical unit with difference in physiological response to (although) the same treatment under the same conditions could explain the high variability in protein expression especially within the individuals of the NE-group. The increased CV rates may possibly be linked to an increase in the quantity of low abundance proteins, which inherently exhibit more variation.

Despite the difference in the number of detected proteins/peptides and observed variation, we can confirm the conservativeness of both proteomics approaches by similarity and high confidence in quantification accuracy of plasma proteome changes brought on by NE due to observed FC matches between DDA and DIA for the common proteins (n=175).

Clearly, the changes in gastrointestinal integrity induced by enteric disease were reflected in the blood proteome of the animals. Performed statistical testing for differential regulation allowed to determine 56 and 59 proteins of distinctive abundance detected with DDA and DIA approach, respectively. The roles of the chicken proteins that we found are of diverse function and localization in the organism. These come from a variety of tissues and include enzymes, receptors, binding proteins, coagulation factors, transport proteins, and more. For four reasons, the chicken plasma proteome we found falls short of a recently published human blood serum proteome of 490 proteins. To gain the insight into the biological processes, molecular functions, and cellular components standing behind the etiology of necrotic enteritis, we conducted the Gene Ontology (GO) analysis on the differentially regulated proteins. Overall, the results suggests that necrotic enteritis leads to significant changes in immune responses (e.g., complement pathway activation, GO:0006958, adaptive immune response, GO:0002250, immune effector process, GO:0002460, etc.), gut integrity (components of collagen structures, GO:0005581), lipid metabolism (fatty acid binding, GO:0005504), and potentially neurological functions (enriched proteins related to synapse pruning, GO:0098883) in broiler chickens. Interestingly, the investigation of changes on the gene level upon NE-infection showed that fatty acid digestion pathways were severely impaired, which may have had an impact on the immunological and metabolic responses of NE-infected birds.

An important attachment target for virulence factors produced by gram-positive bacteria can be collagen, one of the crucial elements of extracellular matrix (ECM) of the host, was found as significantly enriched in this study. Here, authors of a previous study succeeded in identifying NE infection by developing the ELISA-test for detecting collagen adhesin protein (CNA) - the cell wall-anchored protein of C. perfringens, known to be involved in manifestation of the disease. which highlights the relevance of the current finding as well.

Among other significantly enriched proteins categorized into BP-group are proteins of antibacterial function, among which are defensins. Defensins are a group of proteins that are able to significantly influence the innate defensive mechanism of chicken, and possess a featured activity against bacteria, fungi, and viruses. Differentially expressed avian defensin genes in NE-induced chicken lines were discovered in a previous study; the authors relate discovered genes to the host immunological response to NE. Our findings echo the results, which highlights the importance of defensins, and immune response in etiology of NE.

In summary, understanding enriched functions has a potential to provide crucial insights into the molecular processes underlying the disease, which could subsequently aid in developing better strategies for diagnosis and prevention, as well as development of biomarkers of necrotic enteritis/gut health in broilers.

Due to its high sensitivity and throughput, the immunoassays such as enzyme-linked immunosorbent assay (ELISA) are frequently used for the detection, quantification and/or validation of specific proteins in serum or plasma. Despite cost-efficiency and ability to provide the results in a relatively short period of time, the antibody-based methods usually limit the quantification of multiple proteins to only the target one due to employment of specific antibodies. Additionally, the limitation due to availability on the market or costly and time-consuming development, lack of high-quality performing available tests might hamper the transition to the validation phase in biomarker discovery studies. Therefore, an alternative technique for protein detection and quantification such as proteomics serves the purpose and enhances the biomarker discovery pipeline. Our study revealed a positive link between the expression levels of selected proteins (CFD, HPS5, and MASP2) and significant variations in their plasma concentrations between non-infected and infected animals. Moreover, the results also demonstrated a strong agreement between the immunoassay and omics approaches for protein detection. Similarly as previously reported, the proteomics-derived quantification levels could not be fully replicated by ELISA, which was confirmed by a mediocre positive correlation between the methods. Nevertheless, our findings strongly support the application of the omics approach for biomarker discovery in chickens.

### 4. Conclusion

In the present study, we applied a broiler gut inflammation paradigm of necrotic enteritis in order to identify potential blood plasma biomarkers for intestinal health of the chicken. The study found that data independent acquisition (DIA) expressed superior completeness and quantification of the acquired data compared to the more traditional approach of data dependent acquisition (DDA), making DIA well-suited for comparative studies between healthy and diseased samples, as well as for the biomarker discovery purposes. The gut barrier failure in broilers can be diagnosed and evaluated using the prospective biomarkers that have been identified as a basis for field validation as a means of quick and easy diagnostic instruments.

### Example 2: Necrotic enteritis (repeated)

Example 1 was repeated thereby identifying the a more extensive list of blood plasma biomarkers for intestinal health of the chicken. Example 2, however, focused on analysis using the DIA analysing method. Results are shown in Table 6.

**Table 6 Proteins discovered which were significantly different in abundance between control and challenged animals.**

| **Protein.Ids** | **Gene.name s** | **Protein.names** | **Regulation in infected animals** |
|---|---|---|---|
| Q8JIG5 | ORM1 | Alpha-1-acid glycoprotein | Upregulated |
| P21611 | B2M | Beta-2-microglobulin | Upregulated |
| A0A1D5PBP6 | CP | Ceruloplasmin | Upregulated |
| A0A3Q2U7P5 | CFD | Complement factor D | Upregulated |
| A0A1L1RWP3 | DMBT1L | Deleted in malignant brain tumors 1 protein-like | Upregulated |
| A0A1D5NW8 5 | EXFABP | Extracellular fatty acid-binding protein | Upregulated |
| F1N869 | CSF1R | Receptor protein-tyrosine kinase | Upregulated |
| F1NAP7 | MASP2 | Mannan binding lectin serine peptidase 2 | Upregulated |
| F1NPN5 | SPIA3 | Serpin peptidase inhibitor, clade A | Upregulated |
| F1NW65 | HPS5 | Serum amyloid A protein | Upregulated |
| A0A3Q2UGF7 | LOC423629 | Uncharacterized LOC423629 | Upregulated |
| E1C733 | CCL26 | Chemokine | Upregulated |
| R4GGF1 | LRG1 | Leucine rich alpha-2-glycoprotein 1 | Upregulated |
| A0A3Q2UAF0 | APOA4 | Apolipoprotein A4 | Downregulate d |
| P02467 | COL1A2 | Collagen alpha-2(I) chain | Downregulate d |
| F1N869 | SPIA4 | Serpin peptidase inhibitor, clade A | Downregulate d |
| A0A1D5PXP9 | TGFBI | Transforming growth factor-beta-induced protein ig-h3 | Downregulate d |
| F1P256 | ITGBL1 | Integrin subunit beta like 1 | Downregulate d |
| A0A3Q2U321 | POSTN | Periostin | Downregulate d |
| F1NK40 | A2ML4 | Alpha-2-macroglobulin-like 4 | N/A |
| Q6QLQ5 | CATHL1 | Cathelicidin-1 (CATH-1) (Fowlicidin-1) | N/A |
| A0A3Q2TSW 8 | NID2 | Nidogen 2 | N/A |
| P02701 | AVD | Avidin | N/A |
| E1BUA6 | VNN1 | Vanin 1 | N/A |

### Example 3: Dysbiosis

A design of *in vivo* dysbiosis model is described in details in De Meyer et al, 2019. Briefly, a total of 100 day-old broiler chicks were randomly assigned to challenge (ctrl+) and non-challenge (ctrl-) groups, and allocated in a pen-based housing with six birds in each pen, having *ad libitum* feed and water provided. Both groups received commercial feed with rye supplementation (5%) from D12 on. More information regarding diet composition can be found in the above referenced study. To initiate changes into intestinal microbiome, during days 12 to 18 of the experiment the challenge group was administered a combination of antibiotics florfenicol (Flordofen) and enrofloxacin (Enroshort, Dopharma Research B.V.) in concentration 10 mg/kg of body weight (BW) via the drinking water.

Following the course of antibiotics, during three consecutive days each bird of challenge group received orally 1ml of a blend of bacteria known to prevail in the state of intestinal dysbiosis. To promote the damage into enterocytes, chickens were additionally challenges with two coccidial strains *Eimeria acervulina* (6×10⁴oocysts/ml) and Eimeria maxima (4,2×10⁴oocysts/ml) of via oral gavage. A composition of bacterial blend is described in Table 7. Strains of coccidia were produced by Poulpharm BVBA (Izegem, Belgium).

**Table 7 Composition of the bacterial blend for oral inoculation to induce dysbiosis**

| **Strain** | **Culturing conditions** | **Day 19 (CFU/mL)** | **Day 20 (CFU/mL)** | **Day 21 (CFU/mL)** |
|---|---|---|---|---|
| *E. coli* | Luria-Bertani broth (LB, Oxoid) at 37 °C under aerobic conditions | 2.9 ×10¹⁰ | 3.7 ×10⁹ | 3.7 ×10⁹ |
| *L. salivarius* | Man-Rogosa-Sharpe (MRS, Oxoid) medium; microaerophilic conditions (5% O2) | 2.39 ×10⁸ | 3.4 ×10⁸ | 4.58×10¹⁰ |
| *L. crispatus* | | 2.18 ×10⁸ | 3.4 ×10⁹ | 9 · 10×10⁸ |
| *C*. *perfringens* | Brain Heart Infusion (BHI, Sigma, Belgium) broth at 37 °C; 80% N2, 10% CO2 and 10% H2 | 2.41 ×10⁹ | 1 ×10⁹ | 2.46 ×10⁹ |
| *E. faecalis* | Brain Heart Infusion (BHI, Sigma, Belgium) broth at 37 °C | 3.9 ×10¹⁰ | 3.04 ×10¹⁰ | 2.03 ×10¹⁰ |

To assess the level of intestinal permeability, a non-invasive, nontoxic marker iohexol was used as described in the studies of Wilhelm et al. (2020) and Rysman et al. (2023) One day prior to euthanasia the birds (one/pen/group) were administered via oral gavage a solution containing iohexol (Omnipaque 350, GE Healthcare AS, Oslo, Norway) in a concentration of 64.7 mg/kg BW. An hour after administration, blood was collected from the wing vein, left at room temperature to clot and, finally, processed by centrifugation (1,500 × *g,* 15 min). Supernatants (blood serum) were collected, aliquoted and stored -20°C until further analysis.

On day 26, prior to euthanasia blood was withdrawn from all the birds from the jugular vein, after which animals were euthanized with an overdose of pentobarbital, individual body weight was recorded and samples of intestinal tissue and intestinal content were collected. The segments of mid-duodenum, mid-jejunum and mid-ileum were stored in 4% formaldehyde for histological examination; the samples of intestinal content and blood were processed according to the standard procedure as follows: withdrawn blood was kept at 4 °C if could not be processed immediately. To separate plasma, EDTA-treated vacutainers were inverted five times and after processed by centrifugation (1900rcm during 10 min at room temperature, with no brake in the swinging bucket centrifuge). Obtained supernatants were carefully collected into new tubes, omitting disturbing a 'buffy coat'-part of the extract. The plasma samples were aliquoted, snap frozen in liquid nitrogen and stored at - 70°C for further proteomics analysis. Intestinal samples of ileum and colon segments were snap frozen and kept at -70 °C.

Concentrations of iohexol in serum samples detected using ultra-high performance liquid chromatography-tandem mass spectrometry (UHPLC-MS/MS), applying the well-established and validated method for plasma. The morphological parameters i.e. length of the villi, crypt depth and ratio between the two in the tissue samples of the small intestinal were analysed as described in De Maesschalck et al. (2015). Ten villi per section were analysed using a Leica DM LB2 microscope equipped with a camera and a software for image analysis, LAS V4.1 (Leica Application Suite V4, Wetzlar, Germany).

In particular, using the dysbiosis model, the following blood plasma biomarkers for intestinal health of the chicken were identified as shown in Table 8.

**Table 8: biomarkers which are either upregulated or downregulated in animals suffering from dysbiosis (infected animals) compared to healthy animals.**

| **Protein.Ids** | **Gene.name s** | **Protein.names** | **Regulation in infected animals** |
|---|---|---|---|
| F1NAP7 | MASP2 | Mannan binding lectin serine peptidase 2 | Upregulated |
| E1C733 | CCL26 | Chemokine | Upregulated |
| R4GGF1 | LRG1 | Leucine rich alpha-2-glycoprotein 1 | Upregulated |
| A0A1D5PXP 9 | TGFBI | Transforming growth factor-beta-induced protein ig-h3 | Downregulated |
| F1P256 | ITGBL1 | Integrin subunit beta like 1 | Downregulated |
| A0A3Q2U32 1 | POSTN | Periostin | Downregulated |
| P36377 | SPARC | Basement-membrane protein 40 | Downregulated |
| F1NX21 | CD109 | CD109 molecule | Downregulated |
| Q6QLR1 | GAL9 | Gallinacin-9 (Gal-9) (Beta-defensin 9) | Upregulated |
| O42220 | MSTN | Growth/differentiation factor 8 (GDF-8) (Myostatin) | Downregulated |
| P07354 | HAPLN 1 | Hyaluronan and proteoglycan link protein 1 (Cartilage-linking protein 1) | Downregulated |
| A0A1D5P70 3 | MGAM | Maltase-glucoamylase (alpha-glucosidase) | Downregulated |
| A0A1D5PF62 | OVSTL | Ovostatin-like | Downregulated |
| F1P2Y9 | SPINT1 | Serine peptidase inhibitor, Kunitz type 1 | Upregulated |
| P35440 | THBS2 | Thrombospondin-2 | Downregulated |

### Example 4: Mycotoxin - DON

### Feed preparation

The started and grower feed of required composition were produced by Research Diet Services BV (Duurstede, Utrecht, Netherlands). Prior to starting preparation of experimental feed, samples of both types of feed were submitted for multi-toxin liquid chromatography-tandem mass spectrometry (LC-MS/MS) (Primoris, Gent, Belgium) analysis to detect mycotoxin contamination.

DON mycotoxin for the trial was produced in vitro in collaboration with Prof. dr. Labuda Roman (University of Veterinary Medicine, Vienna) using cultures of *F*. *graminearum* cultivated on fermented corn (targeted on DON, with minor traces of ZON (> 5%)). The experimental DON challenge diet was prepared by artificial contamination of the control diet with purified and crystalized DON until final concentration of 10 mg/kg feed was obtained. Experimental feed was mixed with mycotoxin and/or feed additives at the Faculty of Bioscience Engineering in collaboration with Prof. dr. Mia Eeckhout (Department of Food Technology Safety and Health, member of the multidisciplinary research group MYTOX, Ghent University) and analyzed with LC-MS/MS again to confirm expected level of DON (Primoris, Gent, Belgium).

### Trial flow

A total of 200 one-day old broiler chicks vaccinated against Newcastle disease C2 and Infectious Bronchitis (MA5) chicks were purchased from a local hatchery (Vervaeke-Belavi, Tielt, Belgium) and housed in the same animal facility in floor pens on wood shavings. Upon arrival, birds will be randomly divided into four treatment groups:
- a non-DON challenged non-treated group (negative control, n=48),
- a DON-challenged non-treated group (10 mg DON/kg feed, positive control, n=48,),
- a DON-challenged treated group (+ 5 kg Elitox^{®}/Mton of feed, Impextraco, n=48,),
- a DON-challenged treated group (+ 5 kg Elitox^{®}M /Mton of feed, Impextraco, n=48),
maintaining 6 birds in pen (8 pens per treatment).

During the first two days 24h light regime was provided, after- the light/darkness program set to 23/1 during days 3 and 4; finally, 18h/6h schedule of light/darkness program was maintained till D21. Animals had *ad libitum* access to water and feed. Chickens were fed the starter diet until day 10; on day 11 bodyweight was measured individually and the feed was switched to grower until the end of the experiment (D21).

Prior to euthanasia, 5 ml of blood of every animal was collected from a jugular vein into purple-top vacutainers. Blood samples were processed according to the standard procedure as follows: withdrawn blood was kept at 4 °C if could not be processed immediately. To separate plasma, EDTA-treated vacutainers were inverted five times and after processed by centrifugation (1900rcm during 10 min at room temperature, with no brake in the swinging bucket centrifuge). Obtained supernatants were carefully collected into new tubes, omitting disturbing a 'buffy coat'-part of the extract. The plasma samples were aliquoted, snap frozen in liquid nitrogen and stored at - 70°C for further proteomics analysis.

All birds will be euthanized with an overdose of Na-pentobarbital, weighted individually and the following samples will be collected:
- intestinal content from ileum and colon segments (placed in 2ml Eppendorf tubes, snap-frozen in liquid N2 and kept at -20°C);
- small intestinal tissue of duodenum, jejunum, ileum segments (rinsed in phosphate buffered saline (PBS), placed 2ml Eppendorf tubes with *RNAlater*);
- segments of mid-duodenum, mid-jejunum, mid-ileum for sections (placed in the flask with 4% paraformaldehyde)
- organs: bursa of Fabricius, liver, kidneys, spleen (kept in ice until weight measurement).

Intestinal samples of ileum and colon segments, tissue samples were snap frozen and kept at -70 °C. The weight of organs was measured upon collection.

By challenging chicken with the mycotoxin deoxynivalenol (DON), the following blood plasma biomarkers for intestinal health of the chicken were identified as shown in Table 9.

**Table 9: biomarkers which are either upregulated or downregulated in animals challenged with the mycotoxin deoxynivalenol (DON) (infected animals) compared to healthy animals.**

| **Protein.Ids** | **Gene.names** | **Protein.names** | **Regulation in infected animals** |
|---|---|---|---|
| A0A3Q2U5H 6 | SORT1 | Sortilin 1 | Upregulated |
| O93574 | RELN | Reelin | Upregulated |
| A0A3Q2UGB 8 | SELENOP1 | Selenoprotein P1 | Downregulated |
| A0A3Q2U2H 4 | A0A3Q2U2H4_CHIC K | Alpha-2-macroglobulin (results BLASTed) | Downregulated |

### Example 5: use of biomarkers to predict, diagnose and evaluate a treatment of dysbiosis in poultry

In accordance with the present invention, a method for predicting, diagnosing, and treating gastrointestinal diseases and/or disorders in monogastric animals, with a specific emphasis on chickens, is disclosed. The method is based on the identification and analysis of biomarkers that exhibit regulation changes in infected animals compared to healthy animals. These biomarkers, as outlined below, are indicative of dysbiosis-related conditions.

### Step 1: Sample Collection

1.1 Animal Selection: A cohort of chickens from a commercial poultry farm is carefully chosen. These chickens are representative of monogastric animals and are essential to the farm's productivity.

1.2 Sample Collection: Blood samples are meticulously collected from the selected chickens, thereby providing a source of biomarkers indicative of their health status. Plasma is prepared from these blood samples as known in the art.

### Step 2: Biomarker Analysis

2.1 Biomarker Selection: The collected blood plasma samples are subjected to comprehensive analysis for specific biomarkers, the identities of which are listed in Table 8. These biomarkers have been identified based on their significant regulation changes in infected animals compared to healthy animals.

2.2 Quantification: Immunoassay testing (ELISA) is employed to quantify the individual levels of the selected biomarkers. This quantitative analysis provides precise information about the health status of each chicken.

2.3 Comparison to Healthy Levels: The quantified biomarker levels are then meticulously compared to control levels established from healthy chickens, enabling the identification of dysbiosis-related deviations.

### Step 3: Disease Prediction and Diagnosis

3.1 Identification of GI disease/condition: Based on the analysis of biomarker levels, chickens exhibiting substantial upregulation or downregulation of specific biomarkers, as specified in Table 8, are identified as either suffering from dysbiosis or at risk of developing dysbiosis.

### Step 4: Treatment Decision

4.1 Informed Treatment: Upon the identification of chickens with dysbiosis or those at risk, the farm's management and veterinary experts make informed decisions regarding treatment strategies. These interventions may encompass the administration of pharmaceutical compounds or adjustments to dietary and environmental conditions tailored to address the specific gastrointestinal disease and/or disorder associated with dysbiosis.

### Optional Step 5: Post-Treatment Analysis

5.1 Monitoring Biomarker Levels: Following treatment, the biomarker levels in the chickens are re-evaluated. The analysis aims to confirm that the levels of the selected biomarkers have been restored to levels similar to those observed in healthy animals.

By implementing this method, the animal farm can take proactive measures to predict, diagnose, and treat gastrointestinal diseases and disorders in their chicken population, thereby optimizing animal health and ensuring the overall success of their poultry farming operations.

### Example 6: use of biomarkers to predict, diagnose and evaluate a treatment of GI diseases or conditions in pigs

The present invention extends to a method for predicting, diagnosing, and treating gastrointestinal diseases and/or disorders in pigs, with a specific emphasis on the detection and management of gastrointestinal diseases or conditions, which can be prevalent in pig farming.

This method employs a subset of biomarkers chosen from the upregulated biomarkers identified in Table 1, more specifically MASP2, CFD, and HPS5, as they demonstrate significant regulation changes in infected pigs compared to healthy pigs.

### Step 1: Sample Collection

1.1 Pig Selection: A cohort of pigs from a swine farm is thoughtfully selected. Pigs are valuable assets in the swine industry, and their health and well-being are paramount.

1.2 Sample Collection: Blood samples are carefully collected from the selected pigs. Plasma is prepared from these blood samples as known in the art. These samples serve as a source of biomarkers, providing insights into the pigs' health status.

### Step 2: Biomarker Analysis

2.1 Biomarker Selection: The collected blood plasma samples undergo a thorough analysis focused on the quantification of specific biomarkers. In this case, the chosen biomarkers are MASP2, CFD, and HPS5, as they have demonstrated significant upregulation in infected animals, as indicated in Table 1.

2.2 Quantification: Precise laboratory techniques are employed to quantify the levels of MASP2, CFD, and HPS5 in the plasma samples, enabling a quantitative assessment of the pigs' health. In the present case, ELISA is used, however other quantitative methods can be used as well.

2.3 Comparison to Healthy Levels: The quantified biomarker levels are meticulously compared to control levels established from healthy pigs. This comparison allows for the identification of deviations associated with gastrointestinal diseases or conditions.

### Step 3: Disease Prediction and Diagnosis

3.1 Focus on Gastrointestinal Diseases/Conditions: The primary objective of this method is the prediction and diagnosis of gastrointestinal diseases or conditions in pigs. Pigs exhibiting substantial upregulation of MASP2, CFD, and HPS5 biomarkers, as detailed in Table 1, are identified as either suffering from gastrointestinal diseases or conditions or being at high risk of developing such disorders.

### Step 4: Tailored Treatment Strategies

4.1 Informed Interventions: Upon the identification of pigs with gastrointestinal diseases or conditions or those at risk, the swine farm's management and veterinary experts make informed decisions regarding treatment strategies. These interventions may encompass the administration of pharmaceutical compounds or targeted adjustments to dietary and environmental conditions, specifically designed to address the identified gastrointestinal diseases or conditions in pigs.

### Optional Step 5: Post-Treatment Analysis

5.1 Monitoring Biomarker Levels: Following treatment, the biomarker levels in the pigs are re-evaluated. The analysis aims to confirm that the levels of the selected biomarkers have been restored to levels similar to those observed in healthy animals.

Through the implementation of this method, swine farms can proactively predict, diagnose, and treat gastrointestinal diseases or conditions in their pig population, thereby enhancing pig health and ensuring the overall success of their swine farming operations.

### Example 7: use of biomarkers to predict, diagnose and evaluate a treatment of GI diseases or conditions related to mycotoxin DON in pigs and poultry

The following provides a versatile method for predicting, diagnosing, and treating gastrointestinal diseases and/or disorders in both chickens and pigs, acknowledging the significance of gastrointestinal health in these monogastric animals. The method is designed to address gastrointestinal diseases or conditions, which may arise due to the mycotoxin deoxynivalenol (DON), a toxic substance produced by certain moulds, particularly species of Fusarium. DON consumption (through feed contamination) can lead to a range of health issues, including gastrointestinal disturbances, vomiting, diarrhoea, reduced feed intake, and immune system suppression.

### Step 1: Sample Collection for Both Chickens and Pigs

1.1 Animal Selection: A carefully chosen group of chickens and pigs from commercial poultry and swine farms are included in this method. These animals are representative of monogastric species and are pivotal for the success of these farming operations.

1.2 Sample Collection: Blood samples are meticulously collected from the selected chickens and pigs. Plasma is prepared from these samples as known in the art. These samples serve as a source of biomarkers, providing insights into the health status of both animal populations.

### Step 2: Biomarker Analysis for Both Chickens and Pigs

2.1 Biomarker Selection: The collected blood plasma samples from both chickens and pigs undergo comprehensive analysis, with a focus on the quantification of specific biomarkers. The chosen biomarkers are derived from Table 4, which includes biomarkers SORT1 and RELN identified as significantly upregulated in animals challenged with DON compared to healthy animals.

2.2 Quantification: Accurate laboratory techniques are employed to quantify the levels of selected biomarkers. In this case, the biomarkers of interest are A0A3Q2U5H6 (SORT1) and O93574 (RELN), both upregulated as they have demonstrated significant upregulation in animals challenged with DON.

2.3 Comparison to Healthy Levels: The quantified biomarker levels are meticulously compared to control levels established from healthy chickens and pigs, allowing for the identification of deviations associated with gastrointestinal diseases or conditions due to DON exposure.

### Step 3: Disease Prediction and Diagnosis for Both Chickens and Pigs

3.1 Focus on Gastrointestinal Diseases/Conditions: The primary objective of this method is the prediction and diagnosis of gastrointestinal diseases or conditions in both chickens and pigs, especially those arising from DON exposure. Animals exhibiting substantial upregulation specific biomarkers, as detailed in Table 4, are identified as either suffering from gastrointestinal diseases or conditions or being at high risk of developing such disorders.

### Step 4: Tailored Treatment Strategies for Both Chickens and Pigs

4.1 Informed Interventions: Upon the identification of chickens and pigs with gastrointestinal diseases or conditions or those at risk, the respective farm management and veterinary teams make informed decisions regarding treatment strategies. These interventions may encompass the administration of pharmaceutical compounds or targeted adjustments to dietary and environmental conditions, specifically designed to address the identified gastrointestinal diseases or conditions in both chickens and pigs.

### Optional Step 5: Post-Treatment Analysis

5.1 Monitoring Biomarker Levels: Following treatment, the biomarker levels in both chickens and pigs are re-evaluated. The analysis aims to confirm that the levels of the selected biomarkers have been restored to levels similar to those observed in healthy animals.

Through the implementation of this versatile method, poultry and swine farms can proactively predict, diagnose, and treat gastrointestinal diseases or conditions in their animal populations, thereby enhancing animal health and ensuring the overall success of their farming operations.

The present invention is in no way limited to the embodiments described in the examples and/or shown in the figures. On the contrary, methods according to the present invention may be realized in many different ways without departing from the scope of the invention.

## Claims

1. A method for predicting, diagnosing, and/or evaluating a treatment of a gastrointestinal disease and/or disorder in an animal, preferably a monogastric animal such as a chicken, said method comprises the steps of analysing a collected biological sample from said animal for one or more biomarkers chosen from Table 1.

2. The method according to claim 1, wherein individual levels of said one or more biomarkers are quantified.

3. The method according to claim 2, wherein said prediction, diagnosis or evaluation occurs on the basis of said individual levels of said one or more biomarkers.

4. The method according to claim 2 or 3, wherein said quantified biomarker levels are compared to control levels of healthy animals.

5. The method according to any of the previous claims, wherein the sample is a body fluid sample chosen from the group consisting of blood and plasma, preferably plasma.

6. The method according to any of the previous claims wherein said animal is a monogastric animal chosen from the group of pigs, avians species such as chicken, rats, mice, horses, and rabbits, preferably chicken.

7. The method according to any of the previous claims, wherein said markers are protein and/or peptide markers.

8. The method according to any of the previous claims, wherein said analysis occurs by means of an immunoassay.

9. The method according to any of the previous claims, wherein said gastrointestinal disease and/or disorder is caused by a bacterial, fungal, viral, or parasitic infection.

10. The method according to any one of claims 1 to 8, wherein said gastrointestinal disease and/or disorder is caused by a dietary factor or environmental condition.

11. The method according to any of the previous claims, wherein said gastrointestinal disease and/or disorder is necrotic enteritis.

12. A method for evaluating the efficacy of a compound to prevent or treat a gastrointestinal disease and/or disorder in an animal, said method comprises administering to an animal an amount of said compound, and analysing one or more biomarkers chosen from Table 1.

13. The method of claim 12, wherein said method comprises analysing said one or more biomarkers before administering said compound and after administering said compound.

14. A biomarker panel for predicting, diagnosing, and/or evaluating a treatment of a gastrointestinal disease and/or disorder in an animal, preferably a monogastric animal such as a chicken, wherein said biomarker panel comprises at least two markers chosen from Table 1.

15. An immunoassay for predicting, diagnosing, and/or evaluating a treatment of a gastrointestinal disease and/or disorder in an animal, wherein said immunoassay analyses at least two markers chosen from Table 1.

16. Use of a biomarker panel for predicting, diagnosing, and/or evaluating a treatment of a gastrointestinal disease and/or disorder in an animal, preferably a monogastric animal such as a chicken, wherein said biomarker panel comprises one or more biomarkers as listed in Table 1.
